(19) Europäisches Patentamt
European Patent Office
Office européen des brevets

(11) **EP 4 589 337 A1**

(12) **EUROPEAN PATENT APPLICATION**
published in accordance with Art. 153(4) EPC

(43) Date of publication:
**23.07.2025 Bulletin 2025/30**

(21) Application number: **23865575.7**

(22) Date of filing: **13.09.2023**

(51) International Patent Classification (IPC):
*G01S 13/34* (2006.01)    *A61B 5/0245* (2006.01)
*A61B 5/11* (2006.01)    *G01S 13/88* (2006.01)

(52) Cooperative Patent Classification (CPC):
**A61B 5/0245; A61B 5/11; G01S 13/34; G01S 13/88**

(86) International application number:
**PCT/JP2023/033445**

(87) International publication number:
**WO 2024/058226 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.09.2022 JP 2022148633**

(71) Applicant: Kyocera Corporation
Kyoto-shi, Kyoto 612-8501 (JP)

(72) Inventors:
• **KURODA Jun**
Kyoto-shi, Kyoto 612-8501 (JP)

• **SAHARA Tooru**
Kyoto-shi, Kyoto 612-8501 (JP)
• **HOMMA Takuya**
Kyoto-shi, Kyoto 612-8501 (JP)
• **KASHIMA Yuu**
Kyoto-shi, Kyoto 612-8501 (JP)
• **MURAKAMI Youhei**
Kyoto-shi, Kyoto 612-8501 (JP)

(74) Representative: **Viering, Jentschura & Partner
mbB**
**Patent- und Rechtsanwälte
Am Brauhaus 8
01099 Dresden (DE)**

(54) **ELECTRONIC APPARATUS, METHOD FOR CONTROLLING ELECTRONIC APPARATUS, AND PROGRAM**

(57)    An electronic device includes a transmission unit, a reception unit, and a signal processing unit. The transmission unit is configured to transmit a transmission wave. The reception unit is configured to receive a reflected wave of the transmission wave from a target. The signal processing unit is configured to detect a distance, direction, and velocity of the target on the basis of a converted signal obtained by taking the Fourier transform of a beat signal of the transmission wave and the received wave. The signal processing unit is configured to extract, by using a plurality of window functions, signal components corresponding to heart sound vibration associated with the heartbeat of the target from the converted signal. The signal processing unit is configured to perform frequency analysis on an envelope signal obtained by performing envelope processing on the signal components corresponding to the heart sound vibration. The signal processing unit is configured to perform processing to calculate a statistical result on the result of the frequency analysis. The signal processing unit is configured to output time-series data on the frequency of the heartbeat of the target on the basis of the calculated result.

FIG. 2

EP 4 589 337 A1

**EP 4 589 337 A1**

## Description

CROSS-REFERENCE TO RELATED APPLICATIONS

This application claims priority based on Japanese Patent Application No. 2022-148633 filed September 16, 2022, the entire disclosure of which is hereby incorporated by reference.

TECHNICAL FIELD

The present disclosure relates to an electronic device, a method of controlling an electronic device, and a program.

BACKGROUND OF INVENTION

In fields such as the automotive and related industries, for example, technologies for measuring values such as the distance between a vehicle and a given object are gaining importance. In particular, recent years have seen various research into radio detection and ranging (RADAR) technology, which measures values such as the distance to an obstacle or other object by transmitting radio waves, such as millimeter waves, and receiving reflected waves back from the object. The importance of technologies for measuring such distances and the like is expected to increase further with the development of technologies for assisting drivers with driving and technologies related to self-driving, in which driving is partially or fully automated.

Various technologies have been proposed to detect the presence or the like of a given object by receiving reflected waves of transmitted radio waves reflecting off the object. For example, Patent Literature 1 proposes a device that can detect the presence of a person and biological information about the person by using microwaves. As another example, Patent Literature 2 proposes a device that detects vital signs such as the respiration or heartbeat frequency of a living body on the basis of reflected microwave radar signals.

CITATION LIST

PATENT LITERATURE

Patent Literature 1: Japanese Unexamined Patent Application Publication No. 2002-71825
Patent Literature 2: Japanese Unexamined Patent Application Publication No. 2021-32880

SUMMARY

An embodiment of the present disclosure is an electronic device. The electronic device includes a transmission unit, a reception unit, and a signal processing unit. The transmission unit is configured to transmit a transmission wave. The reception unit is configured to receive a reflected wave of the transmission wave from a target. The signal processing unit is configured to detect a distance, direction, and velocity of the target on the basis of a converted signal obtained by taking the Fourier transform of a beat signal of the transmission wave and the received wave. The signal processing unit is configured to extract, by using a plurality of window functions, signal components corresponding to heart sound vibration associated with the heartbeat of the target from the converted signal. The signal processing unit is configured to perform frequency analysis on an envelope signal obtained by performing envelope processing on the signal components corresponding to the heart sound vibration. The signal processing unit is configured to perform processing to calculate a statistical result on the result of the frequency analysis. The signal processing unit is configured to output time-series data on the frequency of the heartbeat of the target on the basis of the calculated result.

An embodiment of the present disclosure is a method for controlling an electronic device. The method involves transmitting a transmission wave from a transmission unit. The method involves a reception unit that receives a reflected wave of the transmission wave from a target. The method involves detecting a distance position, direction, and velocity of the target on the basis of a converted signal obtained by taking the Fourier transform of a beat signal of the transmission wave and the received wave. The method involves extracting, by using a plurality of window functions, signal components corresponding to heart sound vibration associated with the heartbeat of the target from the converted signal. The method involves performing frequency analysis on an envelope signal obtained by performing envelope processing on the signal components corresponding to the heart sound vibration. The method involves performing processing to calculate a statistical result on the result of the frequency analysis. The method involves outputting time-series data on the frequency of the heartbeat of the target on the basis of the calculated result.

An embodiment of the present disclosure is a program. The program causes an electronic device to perform a process. The process involves transmitting a transmission wave from a transmission unit. The process involves a reception unit that receives a reflected wave of the transmission wave from a target. The process involves detecting a distance position, direction, and velocity of the target on the basis of a converted signal obtained by taking the Fourier transform of a beat signal of the transmission wave and the received wave. The process involves extracting, by using a plurality of window functions, signal components corresponding to heart sound vibration associated with the heartbeat of the target from the converted signal. The process involves performing frequency analysis on an envelope signal obtained by performing envelope processing on the signal components corresponding to the heart sound vibration. The process involves performing processing to calculate a statistical result on the result of the frequency analysis. The process involves outputting time-series data on the frequency of the heartbeat of the target on the basis of the calculated result.

BRIEF DESCRIPTION OF THE DRAWINGS

FIG. 1 is a diagram for describing how an electronic device according to an embodiment is used.
FIG. 2 is a function block diagram schematically illustrating a configuration of an electronic device according to an embodiment.
FIG. 3 is a diagram for describing the composition of a signal processed by an electronic device according to an embodiment.
FIG. 4 is a diagram for describing the processing of a signal by an electronic device according to an embodiment.
FIG. 5 is a diagram for describing the processing of a signal by an electronic device according to an embodiment.
FIG. 6 is a diagram for describing the processing of a signal by an electronic device according to an embodiment.
FIG. 7 schematically illustrates an example of the antenna arrangement and the operating principle of an antenna array of an electronic device according to an embodiment.
FIG. 8 illustrates an example of the antenna arrangement in an antenna array of an electronic device according to an embodiment.
FIG. 9 illustrates an example of the processing of a signal by an electronic device according to an embodiment.
FIG. 10 illustrates an example of the processing of a signal by an electronic device according to an embodiment.
FIG. 11 is a flowchart for describing a comparative example of operations by an electronic device according to an embodiment.
FIG. 12 is a flowchart for describing operations by an electronic device according to an embodiment.
FIG. 13 is a flowchart for describing operations by an electronic device according to an embodiment.
FIG. 14 is a diagram for describing multi-window processing by an electronic device according to an embodiment.
FIG. 15 illustrates the relationship between ranks representing an objective signal and a noise signal in singular value decomposition by an electronic device according to an embodiment.
FIG. 16 conceptually illustrates multiresolution analysis using a discrete wavelet transform by an electronic device according to an embodiment.
FIG. 17 is a diagram for conceptually describing processing to classify and discriminate the best heart sound envelope curve by an electronic device according to an embodiment.
FIG. 18 illustrates the result of performing one-dimensionalization processing using the continuous wavelet transform in an electronic device according to an embodiment.
FIG. 19 illustrates an arrangement of time windows for analyzing the frequency of a heart sound envelope curve by an electronic device according to an embodiment.
FIG. 20 illustrates a MUSIC spectrum of a heart sound envelope curve by an electronic device according to an embodiment.
FIG. 21 is a diagram for describing the selection of a heartbeat frequency vector by an electronic device according to an embodiment.
FIG. 22 illustrates a heart sound time-series waveform obtained by an electronic device according to an embodiment.
FIG. 23 illustrates a heart sound time-series waveform obtained by an electronic device according to an embodiment.
FIG. 24 illustrates an example of a heart rate time-series waveform obtained by an electronic device according to an embodiment.
FIG. 25 illustrates an example of the antenna arrangement in an antenna array of an electronic device according to an embodiment.

DESCRIPTION OF EMBODIMENTS

The ability to detect the heart rate of a human body with a good system and high robustness by detecting weak vibrations, such as the heartbeat of the human body, through the transmission and reception radio waves, such as millimeter waves for example, could be useful in a wide variety of fields. An objective of the present disclosure is to provide an electronic device, a method of controlling an electronic device, and a program with which the heart rate of a human body or the like can be detected with good precision and high robustness by transmitting and receiving radio waves. According to an embodiment, it is possible to provide an electronic device, a method of controlling an electronic device, and a program with which the heart rate of a human body or the like can be detected with good precision and high robustness by transmitting and receiving radio waves. The following describes an embodiment in detail, with reference to the drawings.

In the present disclosure, an "electronic device" may be a device driven by electric power. A "user" may be an entity (typically a human being) or an animal that uses a system and/or an electronic device according to an embodiment. The user may include an entity that monitors a human being or other subject by using an electronic device according to an embodiment. The "subject" may be an entity (a human being or an animal, for example) to be monitored using an electronic device according to an embodiment. The user may also include the subject.

In the present disclosure, "heartbeat" may refer to the pulsation of the heart. "Pulsation" may refer to a rhythmic contraction movement performed by the heart.

In the present disclosure, "heart rate" refers to the number of times the heart pulsates in a certain period of time. For example, the heart rate may refer to the number of pulsations per minute. Pulsations occur in the arteries when the heart pumps blood. Accordingly, the number of pulsations by the arteries may also be referred to as the pulse rate or simply the pulse.

In the present disclosure, "heart sound" may refer to the sound of a beating heart. That is, the heart sound may refer to the sound that occurs when the heart contracts and dilates. The "heart sound" may consist of a low, long first sound based on ventricular muscle tension, mitral valve closure, initiation of blood ejection into the arteries, and/or acceleration of blood flow, followed by a high, short second sound derived from aortic valve closure and/or pulmonary valve closure.

In the present disclosure, the heart sound is not necessarily limited to physical sounds based on air vibrations, and may also mean the vibrations themselves caused by the beating (pulsation) of the heart. For example, in the present disclosure, the heartbeat may be assumed to imply a vibrating source, and the heart sound may be assumed to imply the vibrations themselves caused by the vibrating source. In the present disclosure, the heartbeat may also be assumed to imply the heart sound, depending on the situation.

In the present disclosure, the heart sound may refer to the vibrations of the body in association with the movement of the heart. For example, the heart sound may refer to vibrations of the entire body or of the head, neck, chest, throat, arm, leg, wrist, or some other part of the body. For example, the heart sound may refer to vibrations of the skin or the like on the surface of the entire body or of the head, neck, chest, throat, arm, leg, wrist, or some other part of the body. For example, the heart sound may refer to vibrations of clothing, underwear, eyeglasses, or other items worn by a test subject in association with vibrations of the skin or the like on the surface of the entire body or of the head, neck, chest, throat, arm, leg, wrist, or some other part of the body. The heartbeat may refer to the pulsations themselves of the heart. A heartbeat interval, heart rate, or the like may be calculated from the movement of the heartbeat. In the present disclosure, the pulsation of the heart also be referred to as beating.

An electronic device according to an embodiment can detect the heartbeat of a human being or other subject present in the surroundings of the electronic device. Accordingly, anticipated situations in which an electronic device according to an embodiment is used may be, for example, specific facilities or the like used by people engaged in social activities, such as companies, hospitals, nursing homes, schools, sports gyms, and nursing care facilities. For example, it is extremely important for a company to grasp and/or manage the health status of its employees and the like. Similarly, it is extremely important for a hospital to grasp and/or manage the health status of its patients, medical personnel, and the like, and for a nursing home to grasp and/or manage the health status of its residents, staff, and the like. The situations in which an electronic device according to an embodiment is used are not limited to facilities such as companies, hospitals, and nursing homes as described above, and may be any given facility where it is desirable to grasp and/or manage the health status of a subject. The any given facility may also include a non-commercial facility, such as the home of a user. The situations in which an electronic device according to an embodiment is used are not limited to indoors and may also be outdoors. For example, the situations in which an electronic device according to an embodiment is used may also be inside means of transportation such as trains, buses, airplanes, and the like, as well as stations, boarding areas, and the like. The situations in which an electronic device according to an embodiment is used may also be a means of transportation such as an automobile, aircraft, or ship, a hotel, the home of a user, or the living room, bath, toilet, bedroom, or the like in a home.

In a nursing facility, for example, an electronic device according to an embodiment may be used for the purpose of detecting or monitoring the heartbeat of a subject such as a person in need of nursing or care. If an abnormality is found in the heartbeat of a subject such as a person in need of nursing or care, for example, an electronic device according to an embodiment may also issue a predetermined warning to the person in question and/or another person. Consequently, according to an electronic device according to an embodiment, the person in question and/or a staff member of a nursing care facility or the like may recognize that an abnormality is found in the pulse of a subject such as a person in need of nursing or care, for example. On the other hand, if an abnormality is not found (for example, the heartbeat is found to be normal) in the heartbeat of a subject such as a person in need of nursing or care, for example, an electronic device according to an embodiment may also issue a notification to that effect to the person in question and/or another person. Consequently, according to an electronic device according to an embodiment, the person in question and/or a staff member of a nursing care facility or the like may recognize that the pulse is normal for a subject such as a person in need of nursing or care, for example.

An electronic device according to an embodiment may also detect the pulse of an animal other than a human being as the subject. As an example, the electronic device according to an embodiment described hereinafter is described as detecting the pulse of a human being using a sensor based on a technology like millimeter-wave radar.

An electronic device according to an embodiment may be installed in any stationary object, and may also be installed in any moving object. An electronic device according to an embodiment can transmit a transmission wave from a transmitting antenna to the surroundings of the electronic device. An electronic device according to an embodiment can receive, from a receiving antenna, a reflected wave resulting from the transmission wave being reflected. At least one of the transmitting antenna or the receiving antenna may be provided in the electronic device, and may also be provided in a radar sensor, for example.

The following describes a typical example in which an electronic device according to an embodiment is stationary. On the other hand, the subject (human being) whose pulse is to be detected by an electronic device according to an embodiment may be stationary, moving, or moving their body while remaining stationary. Like an ordinary radar sensor, an electronic device according to an embodiment can measure the distance and the like between the electronic device and an object in the surroundings of the electronic device in situations in which the object may move. An electronic device according to an embodiment can measure the distance and the like between the electronic device and an object even if the electronic device and the object are both stationary.

The following describes an electronic device according to an embodiment in detail, with reference to the drawings. First, an example of the detection of an object by an electronic device according to an embodiment will be described.

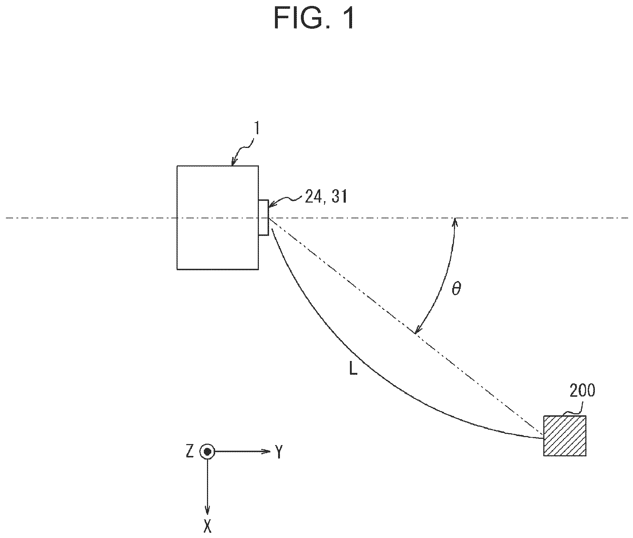

FIG. 1 is a diagram for describing an example of how an electronic device according to an embodiment is used. FIG. 1 illustrates an example of an electronic device provided with the functions of a sensor provided with a transmitting antenna and a receiving antenna according to an embodiment.

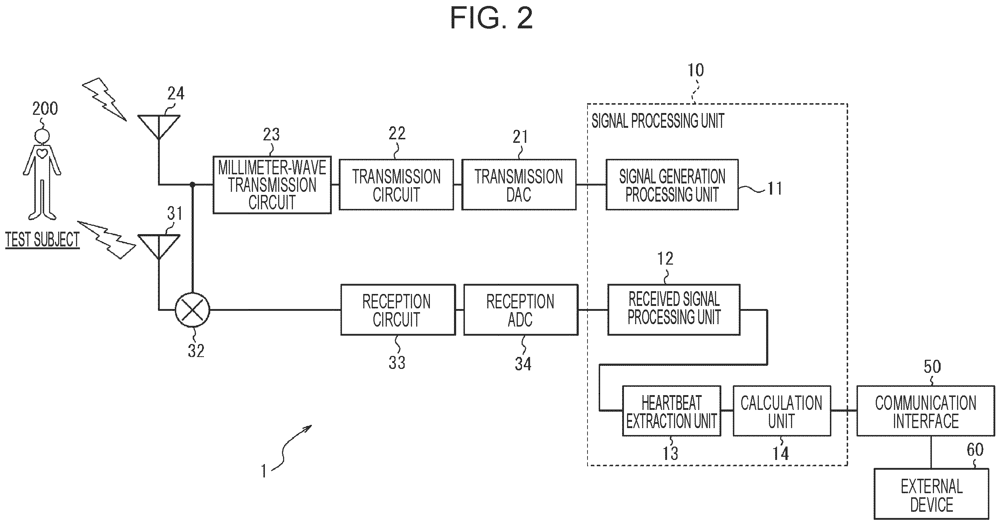

As illustrated in FIG. 1, in an embodiment, an electronic device 1 may be provided with a transmission unit and a reception unit. As described later, the transmission unit may be provided with a transmitting antenna 24. The reception unit may be provided with a receiving antenna 31. Specific configurations of the electronic device 1, the transmission unit, and the reception unit will be described later. For simplicity, FIG. 1 illustrates a situation in which the electronic device 1 is provided with the transmitting antenna 24 and the receiving antenna 31. The electronic device 1 may also include at least one other functional unit, as appropriate, such as at least a portion of a signal processing unit 10 (FIG. 2) included in the electronic device 1. The electronic device 1 may be provided with at least one other functional unit outside the electronic device 1, such as at least a portion of a signal processing unit 10 (FIG. 2) included in the electronic device 1. In FIG. 1, the electronic device 1 may be moving, but may also be stationary without moving.

The example illustrated in FIG. 1 illustrates in a simplified manner the transmission unit provided with the transmitting antenna 24 and the reception unit provided with the receiving antenna 31 in the electronic device 1. The electronic device 1 may also be provided with a plurality of transmission units and/or a plurality of reception units, for example. The transmission unit may be provided with a transmitting antenna 24 formed from a plurality of transmitting antennas. The reception unit may be provided with a receiving antenna 31 formed from a plurality of receiving antennas. The position where the transmission unit and/or reception unit are installed in the electronic device 1 is not limited to the position illustrated in FIG. 1, and may be another position, as appropriate. The number of transmission units and/or reception units may be any number equal to or greater than 1, according to various conditions (or requirements) such as the range and/or precision of heartbeat detection by the electronic device 1.

As described later, the electronic device 1 transmits an electromagnetic wave as a transmission wave from the transmitting antenna 24. For example, if a given object (for example, the subject 200 illustrated in FIG. 1) is present in the surroundings of the electronic device 1, at least a portion of the transmission wave transmitted from the electronic device 1 is reflected by the object to become a reflected wave. Such a reflected wave is then received by the receiving antenna 31 of the electronic device 1, for example, whereby the electronic device 1 can detect the subject as a target.

Typically, the electronic device 1 provided with the transmitting antenna 24 may be a radio detection and ranging (RADAR) sensor that transmits and receives radio waves. However, the electronic device 1 is not limited to a radar sensor. In an embodiment, the electronic device 1 may also be, for example, a sensor based on light detection and ranging (LIDAR) technology, also known as laser imaging detection and ranging, which involves light waves. Sensors such as these can be configured to include a patch antenna or the like. Since technologies such as RADAR and LIDAR are already known, a detailed description may be simplified or omitted, as appropriate. In an embodiment, the electronic device 1 may also be a sensor based on a technology that detects objects by transmitting and receiving sonic or ultrasonic waves, for example.

The electronic device 1 illustrated in FIG. 1 receives from the receiving antenna 31 a reflected wave of a transmission wave transmitted from the transmitting antenna 24. With this arrangement, the electronic device 1 can detect a given subject 200 present within a given distance from the electronic device 1 as a target. For example, as illustrated in FIG. 1, the electronic device 1 can measure the distance L between the electronic device 1 and a given subject 200. The electronic device 1 can also measure the relative velocity between the electronic device 1 and a given subject 200. The electronic device 1 can also measure the direction (angle of arrival $\theta$) from which the reflected wave from a given subject 200 arrives at the electronic device 1.

In FIG. 1, the XY plane may be defined as the plane substantially parallel to the ground, for example. In this case, the positive direction of the Z axis illustrated in FIG. 1 may indicate the vertically upward direction. In FIG. 1, the electronic device 1 may be installed on a plane parallel to the XY plane. In FIG. 1, the subject 200 may be standing on the ground substantially parallel to the XY plane, for example.

The subject 200 may be a human being or the like present in the surroundings of the electronic device 1, for example. The subject 200 may also be a living thing other than a human being, such as an animal, present in the surroundings of the electronic device 1, for example. As described above, the subject 200 may be moving, and may also be stopped or stationary. In the present disclosure, the object to be detected by the electronic device 1 includes inanimate things such as any object, as well as living things such as people, dogs, cats, horses, and other animals. The object to be detected by the electronic device 1 according to the present disclosure may also include a target, including a person, a thing, an animal, or the like, that is detected by radar technology. In the present disclosure, a target may include a person, a thing, an animal, or the like. The following description assumes that the object such as the subject 200 present in the surroundings of the electronic device 1 is a human being (or an animal). Hereinafter, the "subject 200" is also referred to as the "test subject 200", as appropriate. In the present disclosure, the target may also be the subject 200 above.

In FIG. 1, the ratio of the size of the electronic device 1 to the size of the subject 200 does not necessarily indicate the actual ratio. In FIG. 1, the transmitting antenna 24 of the transmission unit and the receiving antenna 31 of the reception unit are illustrated as being installed on the outside of the electronic device 1. However, in an embodiment, the transmitting antenna 24 of the transmission unit and/or the receiving antenna 31 of the reception unit may be installed at various positions in the electronic device 1. For example, in an embodiment, the transmitting antenna 24 of the transmission unit and/or the receiving antenna 31 of the reception unit may be installed inside the electronic device 1, and may not appear on the exterior of the electronic device 1.

The following describes a typical example in which the transmitting antenna of the electronic device 1 transmits radio waves in a frequency band such as millimeter waves (30 GHz or higher) or quasi-millimeter waves (for example, around 20-30 GHz). On the other hand, the transmitting antenna of the electronic device 1 may also transmit radio waves with a frequency bandwidth of 4 GHz, such as from 77 GHz to 81 GHz, for example.

FIG. 2 is a function block diagram schematically illustrating an example configuration of the electronic device 1 according to an embodiment. The following describes an example of the configuration of the electronic device 1 according to an embodiment.

Frequency-modulated continuous-wave radar (hereinafter referred to as FMCW radar) is often used to measure distances and the like using millimeter-wave radar. In FMCW radar, a transmission signal is generated by sweeping the frequencies of the radio waves to be transmitted. Accordingly, in a millimeter-wave FMCW radar that uses radio waves in the 79 GHz frequency band, for example, the frequencies of the radio waves to be used have a frequency bandwidth of 4 GHz, such as from 77 GHz to 81 GHz, for example. Radar in the 79 GHz frequency band is characterized by having a wider usable frequency bandwidth than other millimeter/quasi-millimeter wave radars, such as radars in the 24 GHz, 60 GHz, and 76 GHz frequency bands, for example. The following describes such an embodiment as an example.

The radar scheme of the FMCW radar used in the present disclosure may include a fast-chirp modulation (FCM) scheme that transmits chirp signals on a shorter period than usual. The signal that the electronic device 1 generates is not limited to a signal of the FMCW scheme. The signal that the electronic device 1 generates may also be a signal of any of various schemes other than the FMCW scheme. A transmission signal sequence stored in any storage unit may be different depending on these various schemes. For example, in the case of a radar signal of the FMCW scheme described above, a signal of increasing frequency and a signal of decreasing frequency at each time sample may be used. Known technologies can be applied, as appropriate, for the various schemes described above, and thus a more detailed description is omitted.

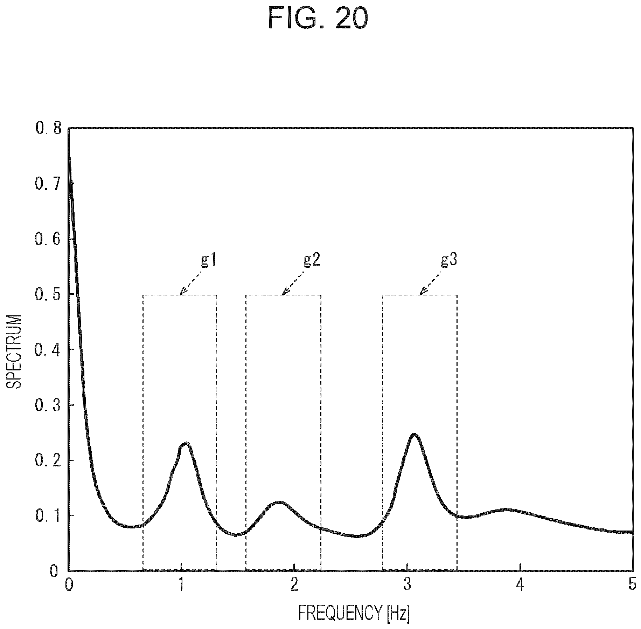

As illustrated in FIG. 2, in an embodiment, the electronic device 1 is provided with a signal processing unit 10. The signal processing unit 10 may be provided with a signal generation processing unit 11, a received signal processing unit 12, a heartbeat extraction unit 13, and a calculation unit 14. The heartbeat extraction unit 13 may execute processing to extract a micro-Doppler component, for example. The heartbeat extraction unit 13 may also execute processing to extract an envelope of the heart sound of the test subject 200. The calculation unit 14 may execute processing to calculate a heartbeat interval (RRI) of the test subject 200, for example. The calculation unit 14 may also execute processing to calculate the heartbeat of the test subject 200, for example. The calculation unit 14 may further execute processing to calculate a heart rate variability (HRV) of the test subject 200, for example. In this case, the calculation unit 14 may also execute processing to perform frequency analysis of time-series data on the extracted heartbeat interval of the test subject 200. The calculation unit 14 may also execute processing to calculate the heart rate variability of the test subject 200 on the basis of frequency analysis of time-series data on the heartbeat interval. In the present disclosure, the calculation unit 14 may calculate the heartbeat interval and then use the heartbeat interval to calculate the heart rate variability. The signal generation processing unit 11, received signal processing unit 12, heartbeat extraction unit 13, and calculation unit 14 will be further described later, as appropriate. In the present disclosure, the heart sound may refer to, for example, a chest vibration waveform (see FIG. 20 and the like) observed directly by radar, or to chest vibrations. The heartbeat refers to the pulsations themselves of the heart. A heartbeat interval, heart rate, or the like may be calculated from the movement of the heartbeat. The heartbeat interval may refer to the time interval between a pulsation of the heart and the next pulsation of the heart.

In an embodiment, the electronic device 1 is provided with a transmission DAC 21, a transmission circuit 22, a millimeter-wave transmission circuit 23, and the transmitting antenna 24 as the transmission unit. In an embodiment, the electronic device 1 is provided with the receiving antenna 31, a mixer 32, a reception circuit 33, and a reception ADC 34 as the reception unit. In an embodiment, the electronic device 1 need not include at least one of the functional units illustrated in FIG. 2, and may also include a functional unit other than the functional units illustrated in FIG. 2. The electronic device 1 illustrated in FIG. 2 may be formed using a circuit configured in basically the same and/or similar way as a common radar using electromagnetic waves in the millimeter-wave band or the like. On the other hand, in the electronic device 1 according to an embodiment, the signal processing by the signal processing unit 10 may include processing different from the processing performed by a common radar of the related art.

In an embodiment, the signal processing unit 10 provided in the electronic device 1 can control operations by the electronic device 1 as a whole, including control of each of the functional units that make up the electronic device 1. In particular, the signal processing unit 10 performs various processing with respect to signals handled by the electronic device 1. To provide control and processing power for executing various functions, the signal processing unit 10 may include at least one processor, such as a central processing unit (CPU) or a digital signal processor (DSP). The signal processing unit 10 may be realized entirely with a single processor, with several processors, or with respectively discrete processors. The processor may be realized as a single integrated circuit. An integrated circuit is also referred to as an IC. The processor may be realized as a plurality of communicatively connected integrated circuits and discrete circuits. The processor may be realized on the basis of any of various other known technologies. In an embodiment, the signal processing unit 10 may be configured as a CPU (hardware) and a program (software) executed by the CPU, for example. The signal processing unit 10 may include a storage unit (memory) required for operations by the signal processing unit 10.

The signal generation processing unit 11 of the signal processing unit 10 generates a signal to be transmitted from the electronic device 1. In the electronic device 1 according to an embodiment, the signal generation processing unit 11 may generate a transmission signal such as a chirp signal (transmission chirp signal). In particular, the signal generation processing unit 11 may generate signals (linear chirp signals) whose frequency varies periodically and linearly. For example, the signal generation processing unit 11 may generate chirp signals whose frequency increases periodically and linearly from 77 GHz to 81 GHz over time. As another example, the signal generation processing unit 11 may generate a signal whose frequency periodically and linearly increases (up-chirp) from 77 GHz to 81 GHz and then decreases (down-chirp) over time. The signal that the signal generation processing unit 11 generates may also be preset in the signal processing unit 10, for example. The signal that the signal generation processing unit 11 generates may also be stored in advance in any storage unit or the like in the signal processing unit 10, for example. Since chirp signals used in technical fields such as radar are already known, a more detailed description is simplified or omitted, as appropriate. The signal generated by the signal generation processing unit 11 is supplied to the transmission DAC 21. For this reason, the signal generation processing unit 11 may be connected to the transmission DAC 21.

The transmission DAC (digital-to-analog converter) 21 functions to convert a digital signal supplied from the signal generation processing unit 11 to an analog signal. The transmission DAC 21 may include a common digital-to-analog converter. The signal converted to analog by the transmission DAC 21 is supplied to the transmission circuit 22. For this reason, the transmission DAC 21 may be connected to the transmission circuit 22.

The transmission circuit 22 functions to convert the signal converted to analog by the transmission DAC 21 to an intermediate frequency (IF) band. The transmission circuit 22 may include a common IF-band transmission circuit. The signal processed by the transmission circuit 22 is supplied to the millimeter-wave transmission circuit 23. For this reason, the transmission circuit 22 may be connected to the millimeter-wave transmission circuit 23.

The millimeter-wave transmission circuit 23 functions to transmit the signal processed by the transmission circuit 22 as a millimeter wave (RF wave). The millimeter-wave transmission circuit 23 may include a common millimeter-wave transmission circuit. The signal processed by the millimeter-wave transmission circuit 23 is supplied to the transmitting antenna 24. For this reason, the millimeter-wave transmission circuit 23 may be connected to the transmitting antenna 24. The signal processed by the millimeter-wave transmission circuit 23 is also supplied to the mixer 32. For this reason, the millimeter-wave transmission circuit 23 may also be connected to the mixer 32.

The transmitting antenna 24 is a plurality of transmitting antennas arranged into an array. In FIG. 2, the configuration of the transmitting antenna 24 is illustrated in a simplified manner. The transmitting antenna 24 transmits a signal processed by the millimeter-wave transmission circuit 23 to the outside of the electronic device 1. The transmitting antenna 24 may include a transmitting antenna array used in a common millimeter-wave radar.

In this way, in an embodiment, the electronic device 1 is provided with a transmitting antenna (transmitting antenna 24) and can transmit a transmission signal (transmission chirp signal, for example) as a transmission wave from the transmitting antenna 24.

As an example, suppose the case in which an object such as the test subject 200 is present in the surroundings of the electronic device 1, as illustrated in FIG. 2. In this case, at least a portion of the transmission wave transmitted from the transmitting antenna 24 is reflected by an object such as the test subject 200. The at least a portion of the transmission wave transmitted from the transmitting antenna 24 that is reflected by an object such as the test subject 200 may be reflected toward the receiving antenna 31.

The receiving antenna 31 receives a reflected wave. The reflected wave may refer to at least a portion of a transmission wave transmitted from the transmitting antenna 24 that is reflected by an object such as the test subject 200.

The receiving antenna 31 is a plurality of receiving antennas arranged into an array. In FIG. 2, the configuration of the receiving antenna 31 is illustrated in a simplified manner. The receiving antenna 31 receives a reflected wave resulting from a transmission wave transmitted from the transmitting antenna 24 being reflected. The receiving antenna 31 may include a receiving antenna array used in a common millimeter-wave radar. The receiving antenna 31 supplies a received signal received as a reflected wave to the mixer 32. For this reason, the receiving antenna 31 may be connected to the mixer 32.

The mixer 32 converts a signal (transmission signal) processed by the millimeter-wave transmission circuit 23 and a received signal received by the receiving antenna 31 to an intermediate frequency (IF) band. The mixer 32 may include a mixer used in a common millimeter-wave radar. The mixer 32 supplies a signal generated as a synthesized result to the reception circuit 33. For this reason, the mixer 32 may be connected to the reception circuit 33.

The reception circuit 33 functions to perform analog processing on a signal converted to an IF band by the mixer 32. The reception circuit 33 may include a common reception circuit that performs conversion to an IF band. The signal processed by the reception circuit 33 is supplied to the reception ADC 34. For this reason, the reception circuit 33 may be connected to the reception ADC 34.

The reception ADC (analog-to-digital converter) 34 functions to convert an analog signal supplied from the reception circuit 33 to a digital signal. The reception ADC 34 may include a common analog-to-digital converter. The signal converted to digital by the reception ADC 34 is supplied to the received signal processing unit 12 of the signal processing unit 10. For this reason, the reception ADC 34 may be connected to the signal processing unit 10.

The received signal processing unit 12 of the signal processing unit 10 functions to perform various processing on a digital signal supplied from the reception DAC 34. For example, the received signal processing unit 12 calculates the distance from the electronic device 1 to an object such as the test subject 200 on the basis of a digital signal supplied from the reception DAC 34 (distance measurement). The received signal processing unit 12 also calculates the relative velocity of an object such as the test subject 200 relative to the electronic device 1 on the basis of a digital signal supplied from the reception DAC 34 (velocity measurement). The received signal processing unit 12 further calculates the bearing angle of an object such as the test subject 200 as seen from the electronic device 1 on the basis of a digital signal supplied from the reception DAC 34 (angle measurement). Specifically, I/Q converted data may be inputted into the received signal processing unit 12. By accepting the input of such data, the received signal processing unit 12 performs a fast Fourier transform (2D-FFT) in each of the distance (range) and velocity directions. The received signal processing unit 12 then performs false alarm suppression and fixed probability conversion by removing noise points through processing such as constant false alarm rate (CFAR). The received signal processing unit 12 performs angle-of-arrival estimation for points that satisfy the CFAR criterion to obtain the position of an object such as the test subject 200. The information generated as a result of the distance measurement, velocity measurement, and angle measurement by the received signal processing unit 12 may be supplied to the heartbeat extraction unit 13.

The heartbeat extraction unit 13 extracts information related to heartbeat from information generated by the received signal processing unit 12. The operations for extracting information related to heartbeat by the heartbeat extraction unit 13 will be further described later. The information related to heartbeat extracted by the heartbeat extraction unit 13 may be supplied to the calculation unit 14.

The calculation unit 14 performs various arithmetic processing, computational processing, and/or the like on information related to heartbeat supplied from the heartbeat extraction unit 13. The arithmetic processing, computational processing, and/or the like by the calculation unit 14 will be further described later. Various information resulting from the arithmetic processing, computational processing, and/or the like by the calculation unit 14 may be supplied to, for example, a communication interface 50. For this reason, the calculation unit 14 and/or signal processing unit 10 may be connected to the communication interface 50. Various information resulting from the arithmetic processing, computational processing, and/or the like by the calculation unit 14 may also be supplied to another functional unit other than the communication interface 50.

The communication interface 50 is configured to include an interface that outputs information supplied from the signal processing unit 10 to, for example, an external device 60. The communication interface 50 may output information on at least one of the position, velocity, and angle of an object such as the test subject 200 as a controller area network (CAN) or other type of signal, for example, to the external device 60 or the like. For example, information on at least one of the position, velocity, and angle of an object such as the test subject 200 may be supplied to the external device 60 or the like via the communication interface 50. For this reason, the communication interface 50 may be connected to the external device 60 or the like.

As illustrated in FIG. 2, in an embodiment, the electronic device 1 may be connected to the external device 60 in a wired or wireless manner via the communication interface 50. In an embodiment, the external device 60 may be configured to include a computer of any kind, a control device of any kind, and/or the like. In an embodiment, the electronic device 1 may be configured to include the external device 60. The external device 60 may be configured in a variety of ways according to the manner in which information on heartbeat and/or heart sound detected by the electronic device 1 is to be used. Accordingly, a more detailed description of the external device 60 is omitted.

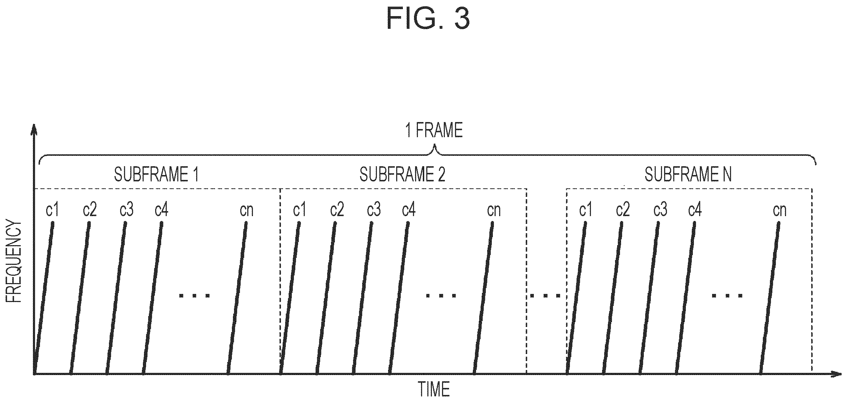

FIG. 3 is a diagram for describing an example of chirp signals generated by the signal generation processing unit 11 of the signal processing unit 10.

FIG. 3 illustrates the temporal structure of one frame in the case of using the fast-chirp modulation (FCM) scheme. FIG. 3 illustrates an example of a received signal of the FCM scheme. FCM is a scheme in which chirp signals illustrated as c1, c2, c3, c4, ..., cn in FIG. 3 are repeated at short intervals (for example, equal to or greater than the round-trip time of electromagnetic waves between the radar and the target as calculated from the maximum ranging distance). In FCM, the transmission and reception processing is often divided into subframe units as illustrated in FIG. 3 for convenient signal processing of a received signal.

In FIG. 3, the horizontal axis represents elapsed time, and the vertical axis represents frequency. In the example illustrated in FIG. 3, the signal generation processing unit 11 generates linear chirp signals whose frequency varies periodically and linearly. In FIG. 3, the chirp signals are indicated as c1, c2, c3, c4, ..., cn. As illustrated in FIG. 3, in each of the chirp signals, the frequency increases linearly over time.

In the example illustrated in FIG. 3, several chirp signals such as c1, c2, c3, c4, ..., cn are included as a single subframe. That is, subframe 1, subframe 2, and so on illustrated in FIG. 3 are each configured to include several chirp signals such as c1, c2, c3, c4, ..., cn. In the example illustrated in FIG. 3, several subframes such as subframe 1, subframe 2, ..., subframe N are included as a single frame (1 frame). That is, the 1 frame illustrated in FIG. 3 is configured to include N subframes. The 1 frame illustrated in FIG. 3 may be frame 1, followed by frame 2, frame 3, and so on. These frames are each configured to include N subframes, in the same and/or similar manner as frame 1. A frame interval of given length may also be included between frames. The single frame illustrated in FIG. 3 may be around 30-50 milliseconds long, for example.

In the electronic device 1 according to an embodiment, the signal generation processing unit 11 may generate a transmission signal as any number of frames. In FIG. 3, some of the chirp signals are omitted from illustration. The relationship between the time and frequency of a transmission signal generated by the signal generation processing unit 11 in this way may be stored in a storage unit or the like of the signal processing unit 10.

In this way, in an embodiment, the electronic device 1 may transmit a transmission signal formed from subframes including a plurality of chirp signals. In an embodiment, the electronic device 1 may transmit a transmission signal formed from frames including a given number of subframes.

The following describes the electronic device 1 as transmitting a transmission signal with a frame structure as illustrated in FIG. 3. However, the frame structure as illustrated in FIG. 3 is an example, and the chirp signals included in one subframes may be of any kind, for example. That is, in an embodiment, the signal generation processing unit 11 may generate subframes including any number of (for example, any plurality of) chirp signals. The subframe structure illustrated in FIG. 3 is also an example, and the subframes included in one frame may be of any kind, for example. That is, in an embodiment, the signal generation processing unit 11 may generate frames including any number of (for example, any plurality of) subframes. The signal generation processing unit 11 may generate signals of different frequency. The signal generation processing unit 11 may generate a plurality of discrete signals each having a different frequency f.

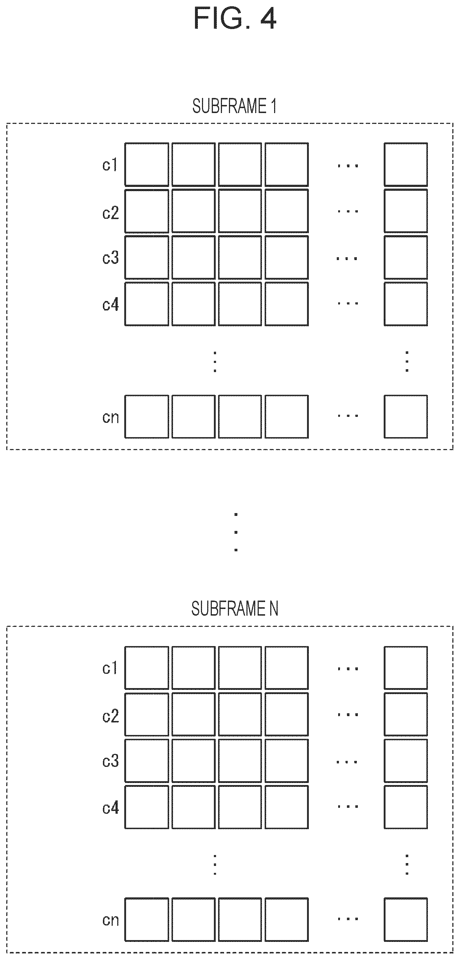

FIG. 4 illustrates another form of a portion of the subframes illustrated in FIG. 3. FIG. 4 is an illustration of samples of a received signal obtained by receiving the transmission signal illustrated in FIG. 3, as a result of performing processing, namely a two-dimensional fast Fourier transform (2D-FFT), in the received signal processing unit 12 (FIG. 2) of the signal processing unit 10.

As illustrated in FIG. 4, each of chirp signals c1, c2, c3, c4, ..., cn is stored in each of subframes such as subframe 1, ..., subframe N. In FIG. 4, each of the chirp signals c1, c2, c3, c4, ..., cn is formed from samples, which are indicated by the horizontally arrayed grid squares. The received signal illustrated in FIG. 4 is subjected to 2D-FFT, CFAR, integration signal processing on each subframe, and/or the like by the received signal processing unit 12 illustrated in FIG. 2.

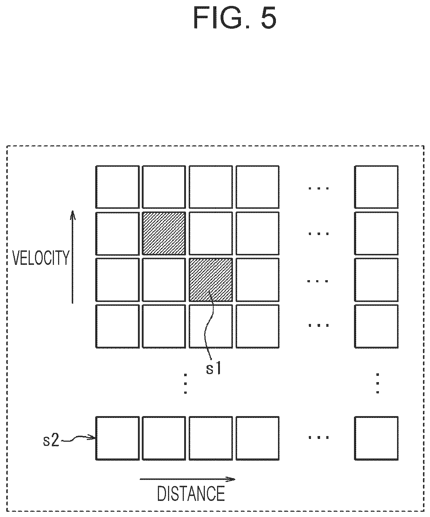

FIG. 5 illustrates an example of a point group in the range-Doppler (distance-velocity) plane calculated as a result of performing 2D-FFT, CFAR, and integration signal processing on each subframe in the received signal processing unit 12 illustrated in FIG. 2.

In FIG. 5, the horizontal direction represents range (distance), and the vertical direction represents velocity. The shaded grid square s1 illustrated in FIG. 5 illustrates a point group indicating a signal exceeding CFAR threshold processing. The non-shaded grid square s2 illustrated in FIG. 5 illustrates a bin (2D-FFT sample) with no point group, which did not exceed the CFAR threshold. Direction estimation is used to calculate the bearing, from the radar, of the calculated point group in the range-Doppler plane illustrated in FIG. 5, and the position and velocity in the two-dimensional plane are calculated as a point group indicating an object such as the test subject 200. Direction estimation may be calculated by a beamformer and/or a subspace method. Typical subspace method algorithms include MUltiple SIgnal Classification (MUSIC) and estimation of signal parameters via rotational invariant techniques (ESPRIT).

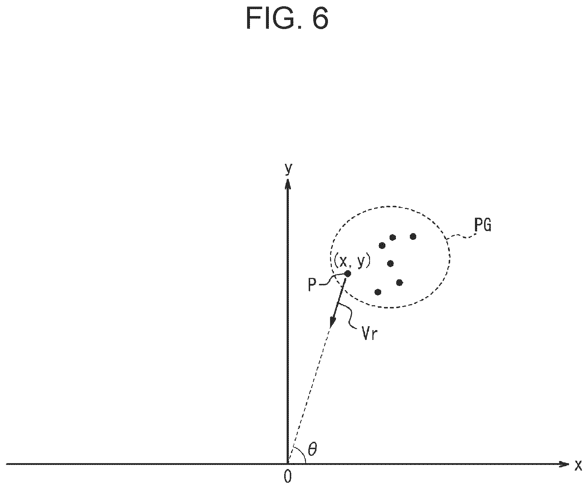

FIG. 6 illustrates an example of the result of the transformation of point group coordinates from the range-Doppler plane illustrated in FIG. 5 to the XY plane by the received signal processing unit 12 after performing direction estimation. As illustrated in FIG. 6, the received signal processing unit 12 can plot a point group PG in the XY plane. The point group PG contains points P. Each of the points P has an angle θ and a radial velocity Vr in polar coordinates.

The received signal processing unit 12 detects an object present in the range where a transmission wave T was transmitted, on the basis of at least one of the 2D-FFT or angle estimation results. The received signal processing unit 12 may perform object detection by performing, for example, clustering processing on the basis of respectively estimated information on distance, information on velocity, and angle information. Known algorithms used in clustering data include density-based spatial clustering of applications with noise (DBSCAN), for example. DBSCAN is an algorithm that performs density-based clustering. In the clustering processing, the average power of the points making up a detected object may be calculated, for example. The information on distance, information on velocity, angle information, and information on power pertaining to an object detected in the received signal processing unit 12 may be supplied to the external device 60 or the like via the communication interface 50, for example.

As above, the electronic device 1 may be provided with a transmitting antenna (transmitting antenna 24), a receiving antenna (receiving antenna 31), and the signal processing unit 10. The transmitting antenna 24 transmits a transmission wave T. The receiving antenna 31 receives a reflected wave R resulting from the transmission wave T being reflected. The signal processing unit 10 detects an object (an object such as the test subject 200, for example) that reflects the transmission wave T, on the basis of the transmission signal transmitted as the transmission wave T and the received signal received as the reflected wave R.

The following further describes direction estimation of an arriving wave by an antenna array of the electronic device 1 according to an embodiment.

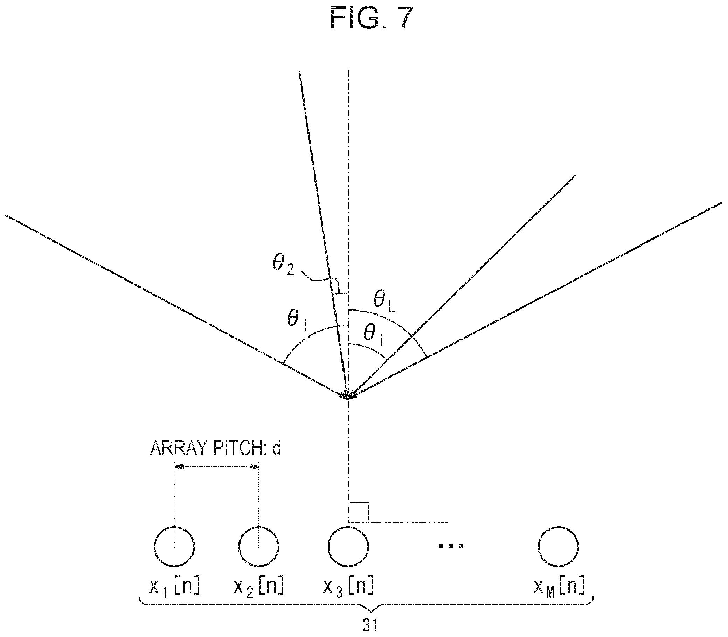

FIG. 7 is a diagram for describing the configuration of the receiving antenna 31 and the principle of direction estimation of an arriving wave by the receiving antenna 31 of the electronic device 1 according to an embodiment. FIG. 7 illustrates an example of the reception of radio waves by the receiving antenna 31.

As illustrated in FIG. 7, the receiving antenna 31 may be a linear arrangement of sensors such as receiving antennas. As illustrated in FIG. 7, in an embodiment, the receiving antenna 31 may be configured to include a plurality of receiving antennas in a linear array. In

FIG. 7, the plurality of antennas $x_1$, $x_2$, $x_3$, ..., $x_M$ that make up the receiving antenna 31 are illustrated by small circles. The receiving antenna 31 may include any plurality of antennas. As illustrated in FIG. 7, the plurality of antennas that make up the receiving antenna 31 are spaced apart from one another by an array pitch d. This type of sensor array, in which sensors (such as antennas, ultrasonic transducers, and microphones) corresponding to various physical waves are disposed in an array, is also referred to as a uniform linear array (ULA). As illustrated in FIG. 7, the physical waves (such as electromagnetic waves and sonic waves) arrive from various directions, such as $\theta_1$ and $\theta_2$, for example. Herein, $\theta_1$ and $\theta_2$ may refer to the angle of arrival described above. In this way, a sensor array like the receiving antenna 31 can estimate the direction of arrival (angle of arrival) by utilizing the phase difference that occurs in measurement values between sensors according to the direction of arrival of a physical wave. This type of technique for estimating the direction of arrival of a wave is also referred to as angle-of-arrival estimation or direction-of-arrival (DoA) estimation.

In the electronic device 1 according to an embodiment, at least one of the transmitting antenna 24 or the receiving antenna 31 may have a plurality of antennas in a linear arrangement. This allows for appropriate narrowing of directivity in the transmission and reception of radio waves in a millimeter-wave radar, for example. When transmitting a transmission wave, the direction of the transmission beam is often controlled by a beamformer. On the other hand, when receiving a reflected wave, the direction of arrival of the reflected wave is more often estimated by subspace methods (such as MUSIC and ESPRIT described above) than by a beamformer. With beamformers and subspace methods, for electromagnetic waves arriving from various directions in the ULA as illustrated in FIG. 7, a phase difference occurs in measurement values between sensors depending on the direction of arrival. Accordingly, this phase difference can be utilized to estimate the direction of arrival of a reflected wave.

The following further describes angle estimation in two directions of an arriving wave by an antenna array of the electronic device 1 according to an embodiment.

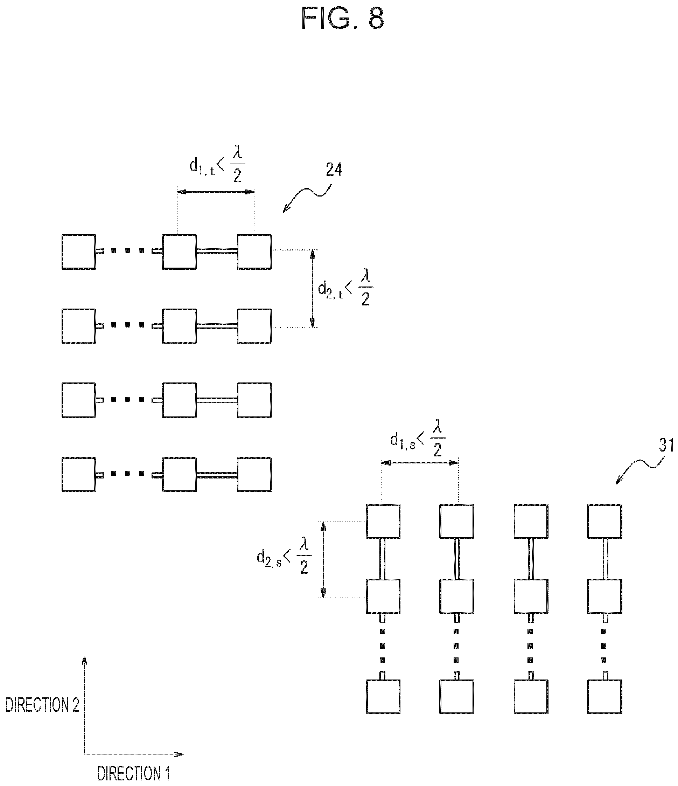

FIG. 8 illustrates an example arrangement of antennas for estimating the direction of arrival with respect to two orthogonal angles.

As illustrated in FIG. 8, in the electronic device 1 according to an embodiment, the transmitting antenna 24 and/or receiving antenna 31 may be configured to include an array of a plurality of patch antenna units.

In the transmitting antenna 24 illustrated in FIG. 8, one patch antenna unit may be configured to include a plurality of elements electrically connected in the direction of direction 1 illustrated in the diagram. In each of the patch antenna units, the plurality of elements may be electrically connected by wiring such as striplines on a board, for example. In each of the patch antenna units, the plurality of elements may be spaced apart from one another by a pitch $d_{1,t}$ shorter than half the wavelength $\lambda$ of the transmission wave. In FIG. 8, each of the patch antenna units may have any number of two or more elements electrically connected.

As illustrated in FIG. 8, the transmitting antenna 24 may include a plurality of patch antenna units arrayed in the direction of direction 2 illustrated in the diagram. The patch antenna units may be spaced apart from one another by a pitch $d_{2,1}$ shorter than half the wavelength $\lambda$ of the transmission wave. In an embodiment, the transmitting antenna 24 may include any number of two or more patch antenna units.

As illustrated in FIG. 8, in an embodiment, the receiving antenna 31 may be a variation of the arrangement of the plurality of elements in the transmitting antenna 24. That is, in the receiving antenna 31 illustrated in FIG. 8, one patch antenna unit may be configured to include a plurality of elements electrically connected in the direction of direction 2 illustrated in the diagram. In each of the patch antenna units, the plurality of elements may be electrically connected by wiring such as striplines on a board, for example. In each of the patch antenna units, the plurality of elements may be spaced apart from one another by a pitch $d_{2,s}$ shorter than half the wavelength $\lambda$ of the transmission wave. In FIG. 8, each of the patch antenna units may have any number of two or more elements electrically connected.

As illustrated in FIG. 8, the receiving antenna 31 may include a plurality of patch antenna units arrayed in the direction of direction 1 illustrated in the diagram. The patch antenna units may be spaced apart from one another by a pitch $d_{1,s}$ shorter than half the wavelength $\lambda$ of the transmission wave. In an embodiment, the receiving antenna 31 may include any number of two or more patch antenna units.

The elements included in the transmitting antenna 24 and the receiving antenna 31 may all be arranged in the same plane (on the surface layer of the same board, for example). The transmitting antenna 24 and the receiving antenna 31 may also be arranged in proximity to each other (monostatic). Direction 1 and direction 2 illustrated in FIG. 8 may be geometrically orthogonal.

With the transmitting antenna 24 and the receiving antenna 31 as illustrated in FIG. 8, the directivity of each of the transmitting antenna and the receiving antenna can be narrowed appropriately. By using the transmitting antenna 24 as illustrated in FIG. 8 to control the direction in which to transmit each transmission wave at each timing for transmitting a transmission wave (transmission signal), a beamformer can be realized for the direction of direction 2 illustrated in FIG. 8. By using the receiving antenna 31 as illustrated in FIG. 8, estimation of the direction of arrival of the reflected wave can be realized for the direction of direction 1 illustrated in FIG. 8. This enables estimation of the direction of arrival of a reflected wave with respect to two substantially orthogonal angles. Accordingly, a point group indicating an object such as the test subject 200 can be acquired three-dimensionally.

The following describes a technique for detecting the heartbeat of the test subject 200 by using the electronic device 1 according to an embodiment.

In an embodiment, the electronic device 1 measures (estimates) the heartbeat of the test subject 200 on the basis of the result of transmitting a millimeter-wave radar or other transmission wave toward the test subject 200 and receiving a reflected wave that was reflected in the chest area of the test subject 200 where the heart resides. As described above, the test subject 200 may be a human being, and may also be an animal. In this case, for example, the vibrations at the position where the test subject 200 is detected to be present by the radar can be filtered by frequency to extract components assumed to be the envelope curve of the heartbeat. Once the components assumed to be the envelope curve of the heartbeat are extracted, the peak-to-peak intervals of the envelope curve can be obtained as the heartbeat interval to roughly calculate the heartbeat interval. The approximation that peaks of the heartbeat envelope roughly correspond to R peaks in an electrocardiogram can be used. Therefore, the "heartbeat interval" may also be referred to as the RR interval (or RRI), like the terms used for electrocardiograms and the like.

The following is an examination of techniques for estimating the heartbeat interval of the test subject 200 from the result of performing the 2D-FFT, CFAR processing, and direction-of-arrival estimation described above and the like. The following describes the manner in which the heartbeat or body motion of a person is expressed as a result of the 2D-FFT performed in FIGs. 4 and 5.

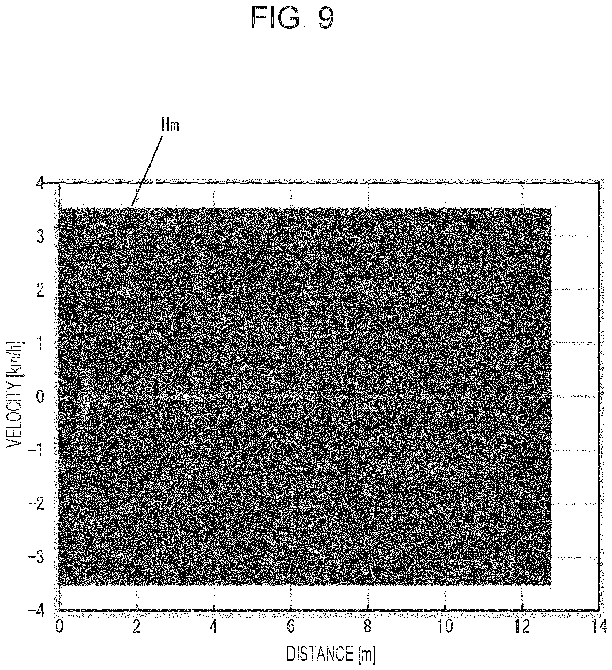

FIG. 9 illustrates an example of the result of receiving and performing 2D-FFT processing on reflected wave of a transmission wave transmitted toward the test subject 200. FIG. 9 illustrates a spectrum illustrating the heart sound and body motion of the test subject 200 as a result of the 2D-FFT. In FIG. 9, the horizontal axis represents distance (range), and the vertical axis represents velocity. In the electronic device 1 according to an embodiment, the signal processing unit 10 (for example, the heartbeat extraction unit 13) may extract a peak Hm as illustrated in FIG. 9, for example, as being body motion such as the heartbeat of the test subject 200. The spectral component indicated by the peak Hm in FIG. 9 includes not only the heart sound of the test subject 200 and the envelope curve of the heart sound, but also body motion. To extract the heartbeat interval, it may be necessary to extract body motion and the like, and thus frequency filtering is assumed to be performed, for example. The frequency filtering performed at this point is assumed to be, for example, a bandpass filter, high-pass filter, and/or low-pass filter targeting frequencies between 0.5 Hz and 10 Hz inclusive.

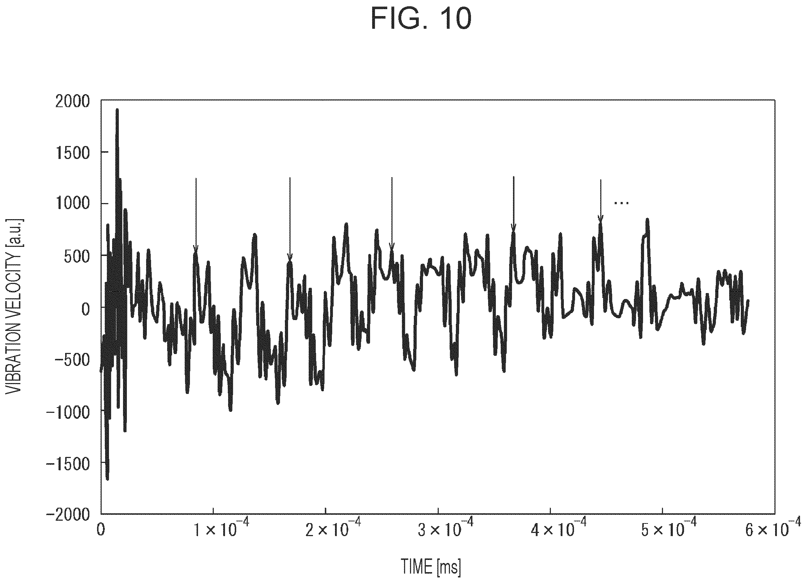

FIG. 10 is a diagram for describing a method of detecting peaks on the basis of the envelope waveform of the heart sound obtained by the frequency filtering described above. The graph illustrated in FIG. 10 illustrates an example of temporal changes in the heart sound envelope waveform of the heart sound extracted by the frequency filtering described above. The envelope waveform illustrated in FIG. 10 includes many peaks as illustrated in the diagram. On the other hand, the beating of the test subject 200 is known to fall within the range approximately from 50 to 130 beats per minute. Therefore, the rough heart sound interval of the test subject 200 can be calculated by selecting, from among the many peaks illustrated in FIG. 10, the peaks having a time interval from 0.4 seconds to 0.8 seconds, that is, the inverse of the number of beats per minute. For example, the peaks indicated by the downward-pointing arrows in FIG. 10 may be selected as the rough heart sound interval of the test subject 200.

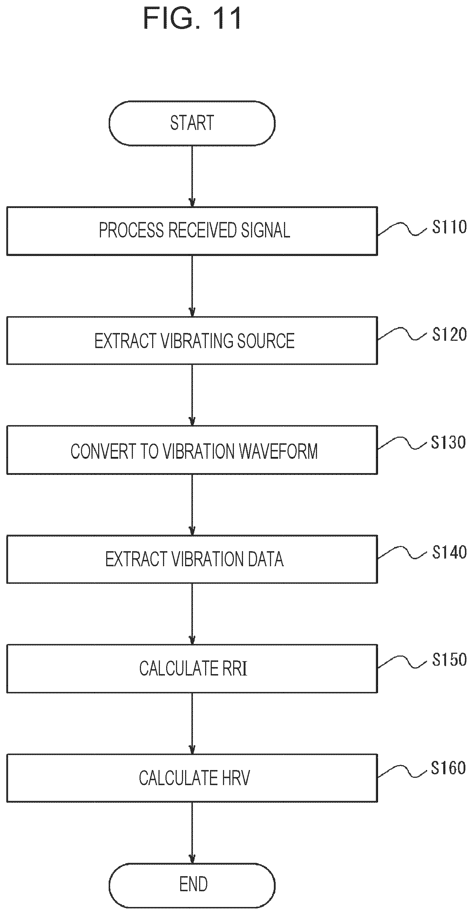

FIG. 11 is a flowchart illustrating an example of the heartbeat interval estimation operations described above. The following refers to FIG. 11 to summarize the heartbeat interval estimation operations described above.

FIG. 11 illustrates operations after the electronic device 1 according to an embodiment has received a reflected wave. That is, the operations illustrated in FIG. 11 presuppose that the electronic device 1 illustrated in FIG. 2 is transmitting a transmission wave (transmission signal) from the transmitting antenna 24. At least a portion of the transmission wave transmitted from the electronic device 1 is reflected by the test subject 200 (the chest portion thereof, for example) to become a reflected wave. The electronic device 1 illustrated in FIG. 2 is receiving such a reflected wave from the receiving antenna 31. From that point, the operations illustrated in FIG. 11 begin.

When the operations illustrated in FIG. 11 begin, in step S110, the signal processing unit 10 of the electronic device 1 processes the signal that is received (received signal). The signal processing performed in step S110 may include the 2D-FFT, CFAR processing, and/or direction-of-arrival estimation described above, for example. Such operations may be performed by the received signal processing unit 12 of the signal processing unit 10, for example.

In step S120, the signal processing unit 10 extracts a vibrating source from the information processed in step S110. The signal processing performed in step S120 may include processing for filtering the data that is the result of performing the 2D-FFT processing, for example. In step S120, the signal processing unit 10 may extract a spectral component at only the position where the test subject 200 is present. The position where the test subject 200 is present may be identified by any of various known techniques. Such operations may be performed by the heartbeat extraction unit 13 of the signal processing unit 10, for example.

In step S130, the signal processing unit 10 converts the processing result of the previous stage into a vibration waveform. The processing performed in step S130 may include processing to extract phase information from IQ data, for example. In step S130, the signal processing unit 10 may include processing to extract vibration data including heart sound from the spectral component of the 2D-FFT processing of the test subject 200 extracted in step S120. Such operations may be performed by the heartbeat extraction unit 13 of the signal processing unit 10, for example.

In step S140, the signal processing unit 10 extracts vibration data from the processing result of the previous stage. The processing performed in step S140 may include frequency filtering, for example. In step S140, the signal processing unit 10 may perform frequency filtering to extract a low-frequency signal including the envelope curve of the heart sound of the test subject 200. Such operations may be performed by the heartbeat extraction unit 13 of the signal processing unit 10, for example.

In step S150, the signal processing unit 10 calculates the RRI interval (RRI) of the test subject 200 by detecting peaks in the beating of the test subject 200 from the processing result of the previous stage and calculating the interval between the peaks. In step S150, the signal processing unit 10 may detect peaks in the low-frequency signal including the envelope curve of the heart sound of the test subject 200. In step S150, the signal processing unit 10 may also calculate and/or extract the interval between peaks. Such operations may be performed by the calculation unit 14 of the signal processing unit 10, for example. As above, in step S150, the signal processing unit 10 may extract the heart sound interval.

In step S160, the signal processing unit 10 may calculate the heart rate variability (HRV) of the test subject 200. In step S160, the signal processing unit 10 may calculate the HRV of the test subject 200 by calculating the spectral density of RRI time-series data. Calculating the power spectral density of RRI time-series data may involve using Welch's method or the like to perform frequency analysis on an RRI time-series waveform. Such operations may be performed by the calculation unit 14 of the signal processing unit 10, for example. In step S160, the signal processing unit 10 may also analyze the spectrum from the processing result of the previous stage.

As above, in an embodiment, the electronic device 1 performs frequency filtering on vibrations at the position where the test subject 200 is present to extract components thought to be the envelope curve of the heartbeat, and obtains the interval between peaks of the envelope curve as the heartbeat interval. In this way, in an embodiment, the electronic device 1 can calculate the rough heartbeat interval of the test subject 200.

In the calculation of the heartbeat interval as described above, several peaks may exist within the timespan of several tens of milliseconds, as illustrated in FIG. 10, for example. Consequently, selecting the peaks of the heartbeat involves some degree of uncertainty. For this reason, the precision of the calculated heartbeat interval will also have an error on the order of several tens of milliseconds. For example, in the calculation of the heartbeat interval described above, an error of around at least 20 ms occurs as compared to the instantaneous RRI acquired by an electrocardiograph. This precision makes it difficult to calculate the heart rate variability (HRV) and perform autonomic nervous system and/or sentiment analysis of a person. That is, in the calculation of the heartbeat interval described above, it is difficult to perform more sophisticated medical analysis by extracting the heart sound. The calculation of the heartbeat interval described above tends to be error-prone if the measurement environment is poor. Accordingly, depending on the measurement environment, obtaining a highly robust pulse rate may be difficult in the calculation of the heartbeat interval described above.

Accordingly, for example, a conceivable approach is to further cut the peaks on the high-frequency side by frequency filtering such that the time interval of the heartbeat is about 0.4 seconds to 0.8 seconds. However, since the precision of the information in the signal illustrated in FIG. 10 is not exceeded, reducing the error to around several tens of milliseconds is considered difficult.

In the calculation of the heartbeat interval described, the heart sound is not actually extracted. Consequently, with the technique described above, obtaining information that would contribute to medical examination (for example, auscultation for diagnosis and treatment) based on the acoustic properties of the heart sound itself is also considered difficult.

One conceivable way of measuring the heart rate of a person or animal with a technology such as radar is to perform frequency filtering on vibrations at the location of a detected target (person or animal) to extract components thought to be the envelope curve of the heartbeat, and then perform frequency analysis on the result. However, to obtain a highly precise and robust heart rate, it may be necessary to search the space of data formed by the radar data appropriately to estimate the heart rate. That is, it may be necessary to estimate the heart rate from the result of extracting chest vibrations corresponding to heart sound from radar data from a plurality of locations (radar ranges), calculate a plurality of heart rate time-series data candidates, and then determine the optimum heart rate data from among the candidates. Chest vibrations corresponding to heart sound may simply be referred to as "heart sound" in the present disclosure. It would be desirable if heart sound could be extracted by radar using a high-frequency band at or above millimeter waves and the heart sound itself could be analyzed to achieve accurate heartbeat interval extraction.

Accordingly, in an embodiment, the electronic device 1 further improves on the technique described above. In an embodiment, the electronic device 1 extracts the heart sound by radar using a high-frequency band at or above millimeter waves, for example, to thereby analyze the heart sound itself and extract an accurate heartbeat interval. In this way, in an embodiment, the electronic device 1 detects the heart rate of a human body or the like with good precision and high robustness by transmitting and receiving radio waves. The following describes such a technique.

In an embodiment, to extract the heart sound of the test subject 200 with high precision, the electronic device 1 performs appropriate signal processing on signals (chirp signals) received by the electronic device 1 in an appropriate sequence to thereby narrow down the subspace and subspace basis in which the signal components of the heart sound of the subject 200 exist. In an embodiment, the electronic device 1 may adopt different basis vectors in the linear space in which the signal of the heart sound of the test subject 200 exists. In this way, in an embodiment, the electronic device 1 may extract subspaces on the basis of coordinates for each while describing the space using an appropriate coordinate system. In an embodiment, the electronic device 1 can use such processing to search for subspaces in which the heart sound of the test subject 200 exists. In an embodiment, an appropriate coordinate system may be used to suit the purpose. For example, coordinate systems such as a coordinate system of the space in which the 2D-FFT processing is performed, a coordinate system of a time-series signal with temporal contraction of the chirp signals, a coordinate system based on a Fourier transform thereof, or a coordinate system based on continuous/discrete wavelets may be used.

In an embodiment, the electronic device 1 may execute characteristic processing like the following on a received signal according to a step-by-step procedure. The following summarizes characteristic processing by the electronic device 1 according to an embodiment. In an embodiment, the electronic device 1 executes two characteristic processes. Namely, in an embodiment, the electronic device 1 executes (1) Subspace extraction and (2) Processing to calculate a time-series vector as heart rate time-series data. The following describes each of these processes in greater detail.

(1) Subspace extraction

In an embodiment, the electronic device 1 executes processing on a received signal to omit data considered unnecessary in a linear space. In an embodiment, the electronic device 1 may execute processing to omit unnecessary subspaces, thereby leaving the necessary subspaces. Specifically, processing like the following may be executed.

(1-1) First stage: dimension reduction

The first stage involves applying an appropriate window function to the 2D-FFT processing of a chirp signal received by the electronic device 1, and using direction-of-arrival estimation to extract only a micro-Doppler component having a point group where the test subject 200 (a person or animal, for example) is present. Multiple window functions are generated around the rough position of the target and centered on a plurality of distance ranges, and these multiple window functions are applied. The processing to apply multiple window functions in this way is hereinafter referred to as "multi-window processing". One time-series signal of vibrations may be generated for one window function, that is, for one central distance range.

(1-2) Second stage: subspace dimension reduction

The second stage involves executing, on a micro-Doppler signal, principal component analysis, singular value decomposition, and/or the like of a set of multiple time-series waveforms of vibrations extracted in the first-stage processing.

(1-3) Third stage: subspace dimension reduction

The third stage involves executing frequency filtering by using at least one of the short-time Fourier transform, the continuous wavelet transform, or a bandpass filter.

(1-4) Fourth stage: subspace dimension reduction

The fourth stage involves extracting the heart sound by performing multiresolution analysis via a discrete wavelet transform, using wavelet and scaling functions appropriate for heart sound.

(2) Processing to calculate a time-series vector as heart rate time-series data

In an embodiment, to facilitate the processing of the extracted heart sound data, the electronic device 1 may execute processing to generate an envelope curve from the waveform of the extracted heart sound. In an embodiment, the electronic device 1 may calculate a time-series vector as heart rate time-series data on the basis of the result of performing frequency analysis on the generated envelope curve. Specifically, processing like the following may be executed.

(2-1) Envelope curve generation

An envelope curve is generated (extracted) from the heart sound extracted in the above section (1) Subspace extraction. The envelope generation processing may use the processing of any of the continuous wavelet transform, a discrete wavelet transform, wavelet scattering coefficients, mel-frequency cepstral coefficients, moving variance, or the like.

(2-2) Frequency analysis of heartbeat

The frequency of the heartbeat is analyzed by applying a window function to the heart sound envelope curve extracted by the processing in the above section (2-1) and performing frequency analysis using fast Fourier transform (FFT) processing or the like. Time-specific frequency characteristics are generated by, for example, the MUSIC algorithm while applying a temporally moving window to the heart sound envelope curve. This generates a multidimensional array in which such time-specific frequency characteristics are superimposed a number of times equal to the multi-windowing described in the above section "(1) Subspace extraction". This multidimensional array can be expressed as a tensor (multidimensional array) like the following.

$$\text{(Peak frequency vector)} \times \text{(Array of time windows)} \times \text{(Multi-window array)}$$

Here, $\times$ represents the tensor product. In the present disclosure, a tensor like the above is also referred to as the heartbeat frequency data tensor.

(2-3) Time-series vector extraction

Dimensionality reduction of the frequency vector and multi-windowing is performed on the basis of the heartbeat frequency data tensor generated by the processing in (2-2) above. This may be used to calculate a time-series vector of the heartbeat frequency and a time-series vector of the heart rate.

According to an embodiment, the electronic device 1 can calculate accurate time-series data of the heart rate by going through the processing in each of the stages described above.

The following describes in greater detail operations by the electronic device 1 according to an embodiment.

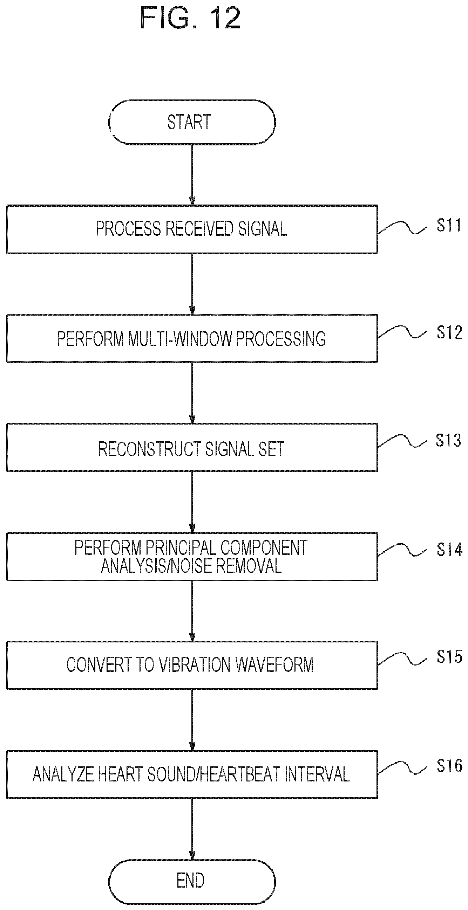

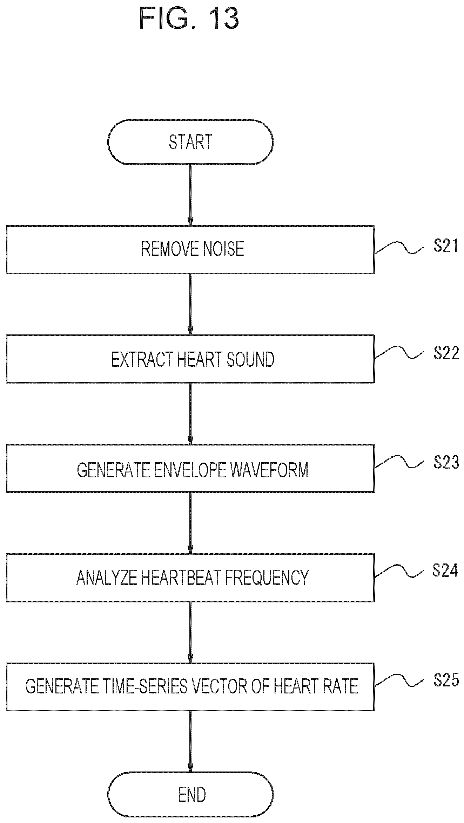

FIG. 12 is a flowchart illustrating an example of operations by the electronic device 1 according to an embodiment. FIG. 13 is a flowchart illustrating in greater detail an example of the operations in step S16 of FIG. 12. The following refers to FIGs. 12 and 13 to describe the flow of operations performed by the electronic device 1 according to an embodiment.

Step S11 illustrated in FIG. 12 can be performed in a manner similar to the operations in step S110 illustrated in FIG. 11. That is, when the operations illustrated in FIG. 12 begin, in step S11, the signal processing unit 10 of the electronic device 1 processes the signal that is received (received signal). The signal processing performed in step S11 may include the 2D-FFT, CFAR processing, and/or direction-of-arrival estimation described above, for example. Such operations may be performed by the received signal processing unit 12 of the signal processing unit 10, for example.

In step S12, the signal processing unit 10 performs multi-window processing. In step S12, the signal processing unit 10 may execute processing to determine candidate clusters of a person or animal and/or filtering processing (multi-window processing) on data processed using the 2D-FFT with multiple window functions applied.

In step S12, the signal processing unit 10 may perform multi-window processing for generating a plurality of heart sound candidates as a stage preceding the selection of the best heart sound candidate. The signal processing unit 10 may perform processing on the data processed using the 2D-FFT to gather a point group corresponding to a person or animal as a cluster. The signal processing unit 10 may perform multi-window processing by applying multiple window functions to a selected cluster. The multi-window processing performed at this point may include processing to generate data processed using the 2D-FFT that will serve as the basis of the plurality of heart sound candidates.

In step S12, the signal processing unit 10 may generate a plurality of heart sounds to serve as candidates by using an appropriate window function to extract only the region where the test subject 200 is present in the range-Doppler plane calculated by the 2D-FFT. In step S12, the signal processing unit 10 may provide a plurality of window functions as the appropriate window function. The signal processing unit 10 may use window functions such as the Hanning window, the Hamming window, and the Blackman-Harris window, for example.

In step S12, the signal processing unit 10 may execute detection of a vibrating source (target) that includes body motion, including the heart sound and/or respiration of the test subject 200, on the basis of the following first to third procedures, for example.

(First procedure)

The signal processing unit 10 classifies point groups exceeding the CFAR threshold in the range-Doppler plane into predefined angular areas on the basis of the result of the direction-of-arrival estimation (see FIGs. 5 and 6). For example, provided that $\theta$ is the angle in the xy plane as illustrated in FIG. 6, angles may be classified into angular areas A to C as follows.

Area A:

$$-10 \text{ deg} < \theta < 10 \text{ deg}$$

Area B:

$$-20 \text{ deg} < \theta \leq 10 \text{ deg}$$

Area C:

$$10 \text{ deg} \leq \theta < 20 \text{ deg}$$

(Second procedure)

The signal processing unit 10 applies clustering to point groups exceeding the CFAR threshold in the range-Doppler plane as indicated by S1 in FIG. 5, from among the groups in a certain one of the angular areas classified by the first procedure. A technique such as DBSCAN may be applied as the clustering, for example.

(Third procedure)

Assuming that L clusters are processed by the second procedure, the signal processing unit 10 compares the deviation $D_{dev}[l]$ in the number of bins in the Doppler direction for the l-th cluster with a certain threshold $D_{dev,th}$. As a result of the comparison, the signal processing unit 10 determines that only those with $D_{dev}[l] \geq D_{dev,th}$ are the test subject 200 and raises a flag HF[l]. That is, the signal processing unit 10 may perform processing as indicated by [Pseudocode 1] below, for example.

$$[\text{Pseudocode 1}]$$
$$\text{for } l = 1 \text{ to } L \text{ do}$$
$$\text{if } D_{dev}[l] \geq D_{dev,th}$$
$$HF[l] = 1$$
$$\text{else}$$
$$HF[l] = 0$$
$$\text{end if}$$
$$\text{end do}$$

In step S13, the signal processing unit 10 reconstructs a set of chirp signals. In step S13, the signal processing unit 10 may reconstruct a set of chirp signals by performing an inverse fast Fourier transform (2D-IFFT) on the plurally generated 2D-FFT data.

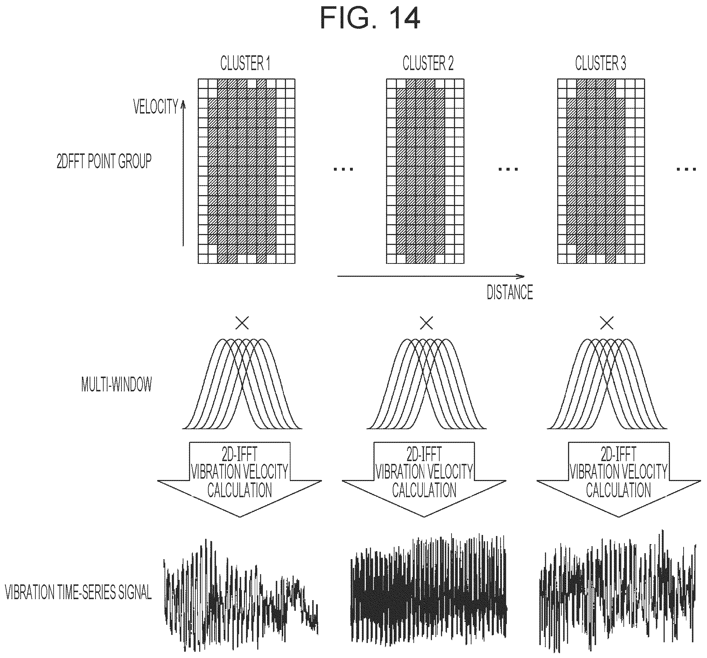

FIG. 14 is a diagram for describing multi-window processing by the electronic device 1 according to an embodiment. FIG. 14 is for describing multi-window processing in which a window function moved in a plurality of range directions is used to generate a plurality of time-series signals from a point group belonging to a cluster selected by the pseudocode 1 described above.
In the upper row of FIG. 14, the horizontal axis direction represents distance (range), and the vertical axis direction represents Doppler velocity. The upper row of FIG. 14 illustrates the results of clustering a point group of 2D-FFT data at a certain time. The upper row of FIG. 14 illustrates how the three clusters of cluster 1, cluster 2, and cluster 3 are generated as a result of clustering. The middle row of FIG. 14 conceptually illustrates how the signal processing unit 10 executes multi-window processing on each of cluster 1, cluster 2, and cluster 3. The middle row of FIG. 14 illustrates how the signal processing unit 10 performs multi-window processing by applying a window function to each of the clusters while shifting the window function a little at a time in the distance (range) direction. The lower row of FIG. 14 illustrates the results of the signal processing unit 10 calculating the vibration velocities by performing the 2D-IFFT on each of cluster 1, cluster 2, and cluster 3. The lower row of FIG. 14 illustrates how a vibration time-series signal is generated by performing the 2D-IFFT on each of the clusters. In actuality, a plurality of vibration time-series signals are obtained for each of the clusters illustrated in FIG. 14 as a result of applying the window function multiple times while shifting the window function a little at a time in the distance (range) direction).
In step S13 of FIG. 11, the signal processing unit 10 applies a plurality of time windows to a cluster formed from a point group selected as having the flag HF[l] = 1 by [Pseudocode 1] described above. This processing results in the generation of 2D-FFT data corresponding to each of the window functions. Let the 2D-FFT data generated by the l-th window function be expressed as in the following expression (1).
[Math. 1]

$$\widehat{S^l} \qquad (1)$$

In this case, in step S13, the signal processing unit 10 can calculate a time-series signal waveform of the vibration velocity according to the following expression (2). The two-dimensional inverse fast Fourier transform (2D-IFFT) is used to reconstruct a set of chirp signals.
[Math. 2]

$$S^l = \mathcal{F}^{-1}(\widehat{S}^l) = [s_1^l, s_2^l, \cdots, s_M^l] \qquad (2)$$

Here, $F^{-1}$ represents the inverse fast Fourier transform, and M represents the number of chirps to be processed by the 2D-FFT.

The following describes an example with only one certain cluster, such as cluster 1 illustrated in FIG. 14.

In step S14, the signal processing unit 10 performs principal component analysis and/or noise removal with respect to the signal. Step S14 may involve singular value decomposition (SVD) as preprocessing to remove noise from the signal. This processing may be performed to remove noise that is low energy and/or low-level noise that is blended in due to the uncertainty of the Fourier transform.

In step S14, the signal processing unit 10 may perform singular value decomposition on the set $S^1$ of received chirp signals extracted in the 2D-FFT plane. If U denotes a matrix of left singular vectors, $\Sigma$ denotes a matrix of singular values arranged diagonally, and V denotes a matrix of right singular vectors, then the singular value decomposition of the set $S^1$ of received chirp signals yields the following expression (3).

[Math. 3]

$$S^l = U\,\Sigma\,V^* \in \mathbb{C}^{N \times M} \qquad (3)$$

In expression (3) above, the * appended to V represents the Hermitian transpose. N indicates the number of samples within a single chirp signal, and M indicates the number of chirp snapshots.

The signal processing unit 10 may limit ($U_{ext}$) the number of row vectors in the rows of left singular vectors in expression (3) above. The signal processing unit 10 may limit ($\Sigma_{ext}$) the number of diagonal elements in the diagonal matrix of singular values in expression (3) above. This removes unwanted noise signals and vibration components other than at the desired position. The resulting signal $S^l_{ext}$ projected into the subspace of the objective signal of $S^l$ is indicated in the following expression (4).

[Math. 4]

$$S_{ext}^l = U_{ext}\,\Sigma_{ext}\,V^* = [s_{ext,1}^l, s_{ext,2}^l, \cdots, s_{ext,M}^l] \in \mathbb{C}^{N \times M} \qquad (4)$$

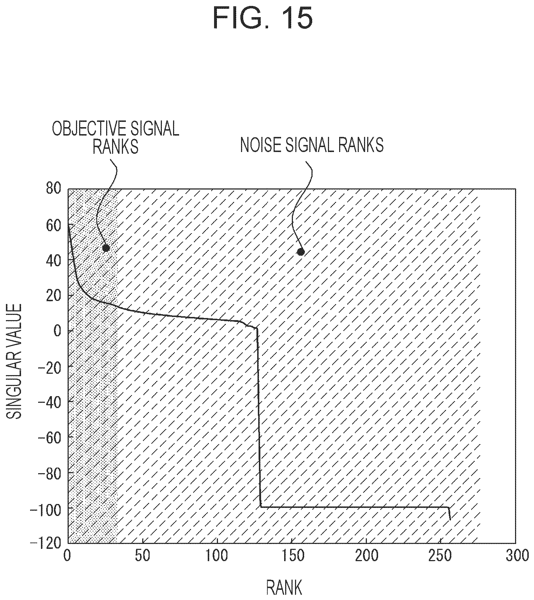

FIG. 15 illustrates the relationship between ranks representing an objective signal and a noise signal in singular value decomposition. FIG. 15 illustrates the SVD results arranged in descending order of singular value (which corresponds to energy), with rank on the horizontal axis and singular value on the vertical axis. The present disclosure adopts the idea that the objective signal is obtained when a signal at or above a certain singular value is extracted. In this case, the rank of the singular value that serves as the boundary between objective signal or not is the portion of the rank region at or below 30 indicated by the dark gray portion of the graph in FIG. 15. As illustrated in FIG. 15, the portion of the rank region at or below 30 is the region of the objective signal. In FIG. 15, the rank portion of the objective signal is between 1 and 30 inclusive. Accordingly, in the present disclosure, singular vectors corresponding to left and right singular values in the rank region between 1 and 30 inclusive may be extracted, for example.

In the example above, the rank portion of the objective signal is between 1 and 30 inclusive. The rank maximum value basically may be determined empirically. The rank maximum value (in the present disclosure, rank 30) may also be determined by a statistical technique. In the graph in FIG. 15, the singular values decrease suddenly at rank 130, and the signals of the ranks thereafter are basically noise. Accordingly, the subsequent rank signals may be unwanted.

In the graph in FIG. 15, the left and right singular vectors (vectors spanning the signal space) corresponding to the singular values of the area from rank 31 to rank 130 also have some objective signal components. These left and right singular vectors are assumed to be mainly due to unnecessary micro-vibrations and/or artificial noise (artifacts) generated by radar signal processing. Consequently, such elements may be cut off.

Once noise is removed by SVD in step S14, the signal processing unit 10 converts the result with the noise removed to a signal waveform (step S15). In step S15, the signal processing unit 10 may convert a beat signal (IQ data) to a vibration time-series signal.

In step S15, the signal processing unit 10 takes the sum in the column vector of each chirp signal on the basis of expression (4) above to obtain the vector indicated in the following expression (5).

[Math. 5]

$$s_{ext,sum}^l = \left[\sum_{m=1}^{M} s_{ext,1}^l, \sum_{m=1}^{M} s_{ext,2}^l, \cdots, \sum_{m=1}^{M} s_{ext,N}^l\right] \qquad (5)$$

In step S15, the argument of $s^l_{ext,sum}$ in the Gaussian plane and the derivative are taken to finally calculate a vector representing the vibration velocity, as indicated in the following expression (6).
[Math. 6]

$$ \boldsymbol{d}^l_{vib} = \arg \boldsymbol{s}^l_{ext,sum}, \quad \boldsymbol{v}^l_{vib} = \frac{d}{dt}\boldsymbol{d}^l_{vib} \qquad (6) $$

In step S16, the signal processing unit 10 uses the obtained plurality of vibration waveforms to extract the heart sound and/or analyze the heartbeat interval. Specifically, in step S16, the signal processing unit 10 extracts the heart sound from the vibration waveforms indicated in expression (6) above. In step S16, the signal processing unit 10 may perform processing to generate a heart sound waveform, processing to calculate the heartbeat interval (RRI), and/or processing to calculate the heart rate variability (HRV). This allows the signal processing unit 10 to calculate the heartbeat interval. This processing may be executed on the plurality of vibration velocity waveforms that correspond to the window functions.

The processing in step S16 illustrated in FIG. 12 as described above may include more specifically at least a portion of the processing in each step from step S21 to step S25 illustrated in FIG. 13. The following describes in greater detail each step from step S21 to step S25 illustrated in FIG. 13.

In step S21 illustrated in FIG. 13, the signal processing unit 10 performs processing (denoising) to remove noise from a time-series waveform. The processing to remove noise may involve empirical Bayes methods, wavelet techniques such as the continuous wavelet transform (CWT), or the like.

More specifically, in step S21, the signal processing unit 10 performs preprocessing to further remove unwanted noise from the vibration velocity vector $v^l_{vib}$ that corresponds to the l-th window function.

In step S21, the signal processing unit 10 may perform noise removal processing through band limiting using empirical Bayes methods and/or continuous wavelets. In step S21, the signal processing unit 10 may also perform noise removal processing through frequency subtraction using a noise profile for artifact noise and the like associated with the nonlinear processing from step S11 to step S14 illustrated in FIG. 12.

In step S22, the signal processing unit 10 extracts the waveform of the objective signal, that is, the waveform of the heart sound. In this case, for example, a technique involving discrete wavelets such as the maximal overlap discrete wavelet transform (MODWT) may be used.

More specifically, in step S22, the signal processing unit 10 may employ a wavelet waveform with a waveform similar to a heart sound waveform in the signal denoised as preprocessing, and execute multiresolution analysis using a discrete wavelet transform. In this way, in step S22, the signal processing unit 10 empirically extracts only the subspace at the level of multiresolution analysis where the heart sound of the test subject 200 is present. In this way, the signal processing unit 10 may extract the heart sound of the test subject 200 in step S22. Specifically, to improve the temporal resolution, the signal processing unit 10 may use maximal overlap multiresolution analysis (MODWT) or the like. Appropriate wavelet bases for heartbeat extraction may be symlet and Daubechies wavelets, for example. The order of the wavelets may be set case by case, as appropriate.

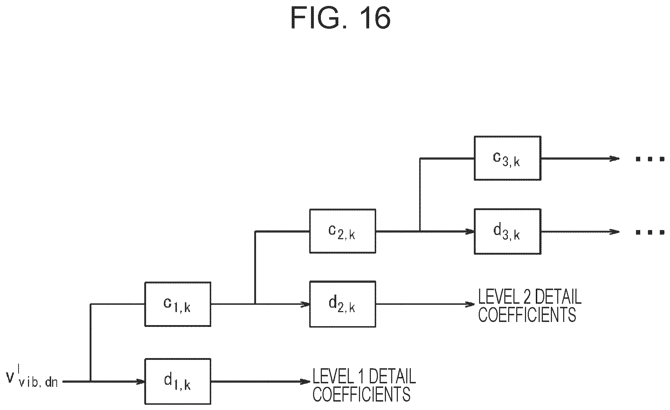

Provided that $v^l_{vib,dn}$ is the signal resulting from performing the denoising process in step S21 on the signal velocity vector $v^l_{vib}$, the multiresolution analysis illustrated in FIG. 16 may be performed on the real part $Re(v^l_{vib,dn})$. FIG. 16 conceptually illustrates multiresolution analysis using a discrete wavelet transform. The signal processing unit 10 can obtain a heart sound waveform h by reconstruction with the empirically appropriate levels limited to $j_0 \in N$ in the following expression (7).
[Math. 7]

$$ \boldsymbol{h}^l = \frac{1}{\sqrt{M}}\left[\sum_{k=1}^{M-1} c_k \boldsymbol{\phi}_{j_0,k} + \sum_{j=j_1}^{j_0}\sum_{k=1}^{M-1} d_{j,k} \boldsymbol{\psi}_{j,k}\right] \in \mathbb{R}^M \qquad (7) $$

Here, $\Phi_{j0,k}$ and $\Psi_{j,k}$ represent a scale function and a wavelet function, respectively, and $c_k$ and $d_{j,k}$ represent a coefficient of each.

In this way, in an embodiment, the electronic device 1 can obtain a heart sound waveform h like the waveforms illustrated in FIGs. 26 and 27 described later, for example. The result can be utilized directly in heart sound diagnosis, treatment, and the like.

In step S23, the signal processing unit 10 generates an envelope waveform. The envelope waveform generation processing may use the processing of any of the continuous wavelet transform, a discrete wavelet transform, wavelet scattering coefficients, mel-frequency cepstral coefficients, moving variance, or the like. In step S23, the signal processing unit 10 calculates a scalogram using the continuous wavelet transform and extracts an envelope waveform by one-dimensionalization thereof to detect (extract) energy peaks in the objective signal (heart sound waveform) extracted by step S22. At this point, the discrete/continuous wavelet transform, moving variance, Hilbert transform, and/or the like may be used.

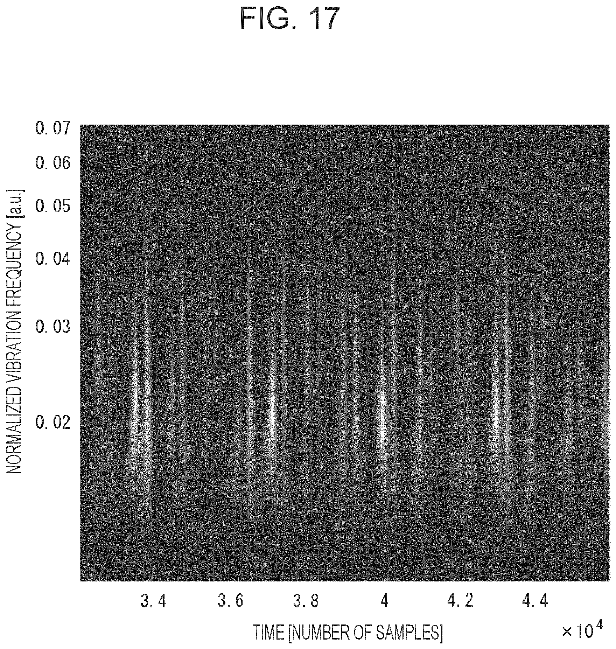

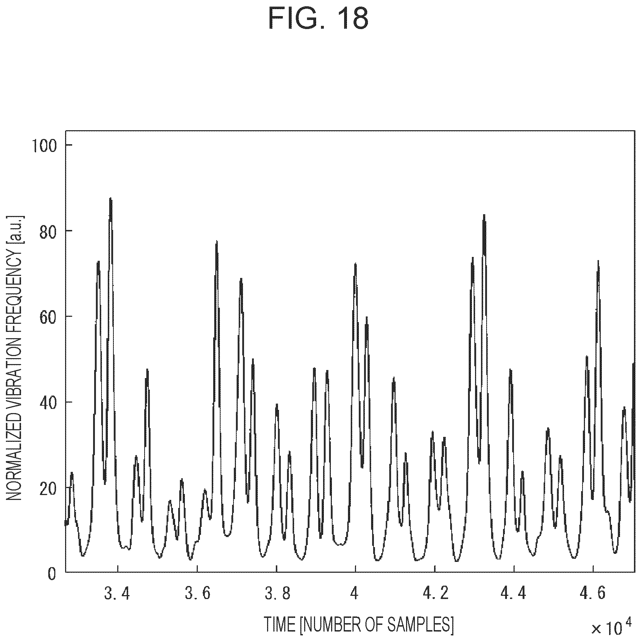

More specifically, in step S23, the signal processing unit 10 may obtain a scalogram using the continuous wavelet transform to obtain an envelope waveform of the heart sound waveform $h^l$ indicated in expression (7) above. The signal processing unit 10 takes the sum over a certain range from $f_1$ to $f_2$ on the frequency axis at each time in the scalogram. The signal processing unit 10 thus obtains a one-dimensional waveform $s^l_h$, as illustrated in FIGs. 17 and 18. FIGs. 17 and 18 are diagrams illustrating examples of a continuous wavelet transform analysis result. The continuous wavelet transform may be replaced by a discrete wavelet transform with the resolution set appropriately.

FIG. 17 illustrates a scalogram of the heart sound waveform $h^l$. FIG. 17 illustrates change over time in the frequency of normalized vibrations. The horizontal axis of FIG. 17 represents time in units of the number of samples, and the vertical axis of FIG. 17 represents the frequency of vibration normalized for each such unit of time.

FIG. 18 illustrates the result of performing one-dimensionalization processing using the continuous wavelet transform on the scalogram of the heart sound waveform $h^l$ illustrated in FIG. 17. FIG. 18 illustrates change over time in the vibration velocity. The horizontal axis of FIG. 18 represents time in units of the number of samples, and the vertical axis of FIG. 18 represents vibration velocity.

The matrix representing the absolute values of the scalogram is assumed to satisfy the following expression (8). [Math. 8]

$$\boldsymbol{S}_h \in \mathbb{R}^{M \times L} \qquad (8)$$

In this case, the one-dimensionalized envelope waveform $e^l_h$ is represented as in the graph illustrated in FIG. 18. The signal processing unit 10 can calculate the envelope waveform $e^l_h$ according to [Pseudocode 2] below.

[Pseudocode 2]

for m=1:M

[Math. 9]

$$\boldsymbol{e}^l_h = \sum_{f_1 \le f \le f_2} \boldsymbol{S}_h \in \mathbb{R}^M \qquad (9)$$

end

In step S24, the signal processing unit 10 analyzes the frequency of the heartbeat. The number of envelope waveforms (heart sound envelope curve data) generated by the processing in step S23 is equal to the number of window functions, as illustrated in FIG. 14. The following illustrates frequency analysis with respect to one of the plurality of window functions.

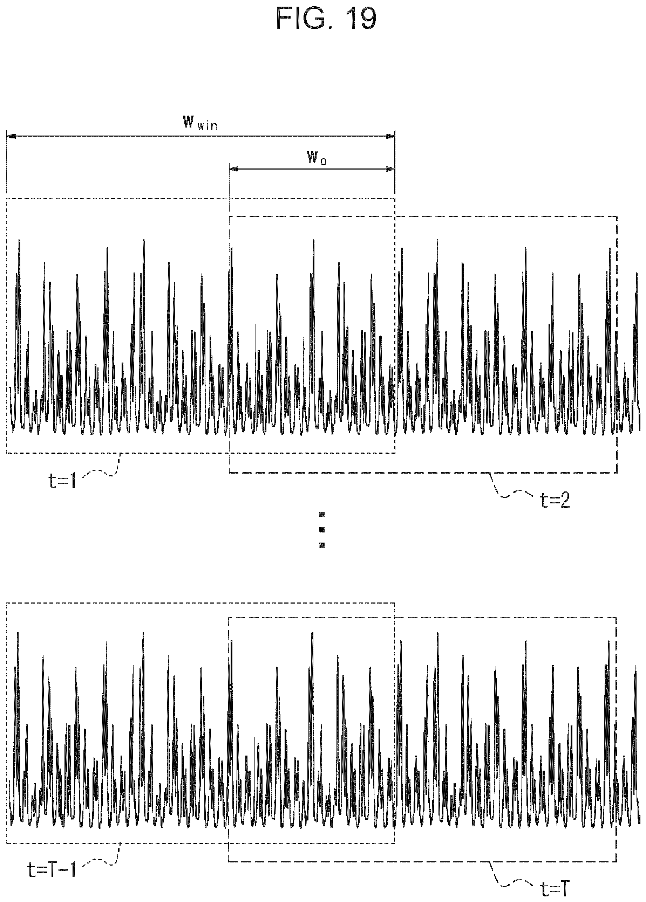

FIG. 19 illustrates an arrangement of time windows for analyzing the frequency of a heart sound envelope curve by the signal processing unit 10. In the heart sound envelope curves illustrated in the upper part of FIG. 19, time windows for extracting the time frames t = 1 and t = 2 are illustrated. In the heart sound envelope curves illustrated in the lower part of FIG. 19, time windows for extracting the time frames t = T-1 and t = T are illustrated. Here, T and t are discrete times, each indicating a non-negative integer. In the heart sound envelope curves illustrated in the upper part of FIG. 19, the width $w_{win}$ of the time window is illustrated along with the width $w_o$ by which the time windows overlap one another. The moving step of the time window is $w_{step} = w_{win} - w_o$.

In general, heart rate is very low frequency. Consequently, analysis of the frequency may necessitate the use of high-resolution techniques. The following describes the MUltiple SIgnal Classification (MUSIC) algorithm as one of the subspace methods. The following describes processing to perform frequency analysis using MUSIC. This processing may include the following [First processing] through [Fourth processing].

[First processing]

The signal processing unit 10 generates an observation matrix $X = [x_1, x_2, ..., x_N]$ by arranging time-series signals by snapshots. The observation matrix X can be obtained by dividing the samples in one of the windows illustrated in FIG. 19 into snapshots of equal size.

[Second processing]

The signal processing unit 10 obtains an autocorrelation matrix $R_x = 1/N \cdot X \cdot X^H$ on the basis of the observation matrix generated in [First processing] above. The appended superscript H indicates the Hermitian transpose.

[Third processing]

The signal processing unit 10 eigendecomposes the autocorrelation matrix Rx obtained in [Second processing] above. In the eigendecomposition, the signal processing unit 10 classifies eigenvectors with large eigenvalues as basis vectors of a signal space, and classifies eigenvectors with small eigenvalues as basis vectors of a noise space. The eigenvectors are sorted in descending order of eigenvalue, the $(1 \leq m \leq p)$-th eigenvectors are obtained as the signal space, and the $(p+1 \leq m \leq M)$-th eigenvectors are obtained as the basis vectors $v_m(p+1 \leq m \leq M)$ of the noise space.

[Fourth processing]

The signal processing unit 10 takes the inner product of the basis vectors of the noise space with respect to a frequency vector $e = [1, e^{j\omega}, e^{j2\omega}, ..., e^{j(M-1)\omega}]^T$ for the angular frequency $\omega$. In this way, the MUSIC spectrum indicated in the following expression (10) can be obtained.
[Math. 10]

$$P_{MU}(\omega) = \frac{1}{\sum_{m=p+1}^{M} |e^H v_m|^2} \qquad (1\ 0)$$

FIG. 20 illustrates an example of a calculated MUSIC spectrum (frequency analysis) computed according to expression (10) above. The vertical axis of FIG. 20 represents MUSIC spectrum, and the horizontal axis of FIG. 20 represents frequency. In the MUSIC spectrum illustrated in FIG. 20, there are three frequency peaks, indicated as g1, g2, and g3. The frequency peaks g1, g2, and g3 are at approximately 1 Hz, approximately 1.7 Hz, and approximately 3.1 Hz, respectively.
As a result of the processing like the above, frequencies appropriate as the heartbeat frequency may be finally picked up. The processing of picking up frequencies appropriate as the heartbeat frequency may be performed by the processing in the following two stages. These stage of processing may be performed by the signal processing unit 10 in the electronic device 1 according to an embodiment.

[Processing A]

A plurality of heartbeat frequency ranges to be detected are set. Frequency peaks within each of the frequency ranges are extracted. A time-series vector of the frequency peaks is generated for each of the heartbeat frequency ranges to be detected.
In general, the normal human heart rate is about 40 to 160 beats per minute (BPM = 40-160). Consequently, the range of human heart rate is divided into three classes of 40 beats each as follows.

Class 1: BPM = 40-80
Class 2: BPM = 80-120
Class 3: BPM = 120-160

If the BPM is converted to frequency in each of these classes, the classes of heartbeat frequency are as follows.

Class 1: 0.67 [Hz] - [1.33] Hz
Class 2: 1.33 [Hz] - [2.0] Hz
Class 3: 2.0 [Hz] - [2.67] Hz

Accordingly, from among the frequency peaks in the MUSIC spectrum as illustrated in FIG. 20, the peaks corresponding to Class 1, Class 2, and Class 3 described above are extracted in individual time frames. For example, the frequency of the peak g1 illustrated in FIG. 20 is approximately 1 Hz and thus corresponds to Class 1. The frequency of the peak g2 illustrated in FIG. 20 is approximately 1.7 Hz and thus corresponds to Class 2. The frequency of the peak g3 illustrated in FIG. 20 is approximately 3.1 Hz and thus does not correspond to any of the classes. The above results are summarized as follows.

Class 1: peak g1
Class 2: peak g2

Class 3: N/A

The processing above can be expressed by [Pseudocode 3] below. Let T be the number of time windows (time frames) to be processed, and let t be an index thereof. Let J be the number of heartbeat frequency classes, and let j be an index thereof. Let $f_j^{min}$ and $f_j^{max}$ be the minimum and maximum values, respectively, of the frequency in the heartbeat frequency class j. Let L be the number of window functions on the 2D-FFT illustrated in FIG. 14, and let l be an index thereof. Let $p_{MU,peak}$ be a vector of the peaks of the MUSIC spectrum $P_{MU}(\omega)$ in time frame t. Let $F_{l,t,j}$ be the heartbeat frequency data tensor to be obtained.

[Pseudocode 3]

for l=1:L

  for t=1:T

    for j=1:J

[Math. 11]

$$\boldsymbol{F}_{l,t,j} = \text{find}\left(f_j^{min} \leq \boldsymbol{p}_{MU,peak} \leq f_j^{max}\right) \qquad (1\ 1)$$

end

end

end

In expression (11) above, find(Condition) is a function that finds a value in $p_{MU,peak}$ that falls within the maximum value and the minimum value. If such a value is not found, NaN (Not a number; standardized in the IEEE 754 floating point standard) or the like may be specified to clearly indicate that such a value does not exist.

[Processing B]

The optimum vector is selected from among the frequency peak time-series vectors generated for the detected plurality of heartbeat frequency ranges, and this optimum vector is obtained as the final heartbeat frequency vector.
Processing is performed to set the indices l and j of the calculated heartbeat frequency data tensor $F_{l,t,j}$ to one natural number. That is, processing is performed to select the best vector from among the L×J heartbeat frequency vectors. This processing is achieved by selecting the frequency vector that meets the following conditions: (1) few missing values (NaNs); and (2) the least change in heartbeat frequency.
Condition (1) is based on the principle that if there are many missing values, there will be no peak in the MUSIC spectrum that corresponds to Class j. Condition (1) is based on the principle that if the position of the window on the 2D-FFT and the heartbeat frequency class are not appropriate, the peak frequencies in the MUSIC spectrum will not settle and have extreme variations.

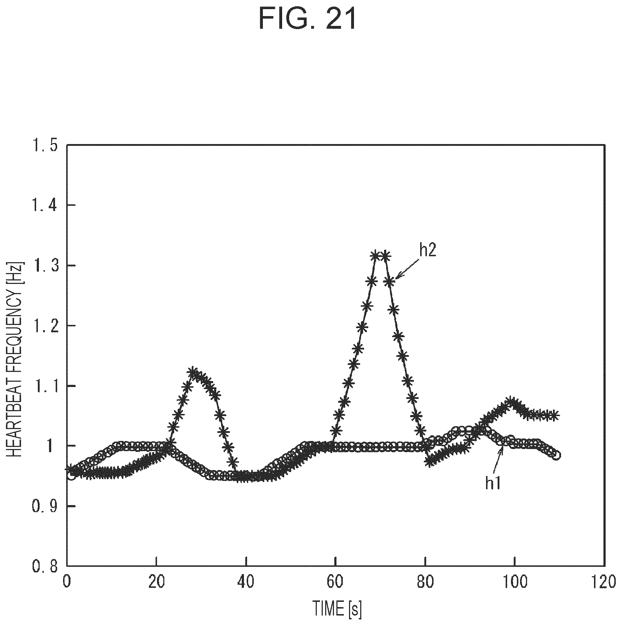

FIG. 21 is a diagram for describing the selection (quality determination) of a heartbeat frequency vector by the electronic device 1 according to an embodiment. The vertical axis of FIG. 21 represents heartbeat frequency, and the vertical axis of FIG. 21 represents time. In FIG. 21, the graph h1 illustrates an example of a heartbeat frequency vector judged to be the optimum by the electronic device 1 according to an embodiment. In FIG. 21, the graph h2 illustrates an example of a heartbeat frequency vector judged to be inappropriate by the electronic device 1 according to an embodiment. As seen in the graph h2 in FIG. 21, a heartbeat frequency vector calculated on the basis of an inappropriate window position on the 2DFFT and/or an inappropriate heartbeat frequency class causes unnaturally large variations.
In an embodiment, the electronic device 1 can take the variance, for example, to statistically selected a heartbeat frequency vector like the graph h1 and/or the graph h2 illustrated by way of example in FIG. 21. In this case, conditional expression for selection can be written as in the following expressions (12) and (13), for example. Expression (12) indicates condition (1) above. Expression (13) indicates condition (1) above.
[Math. 12]

$$\left(\hat{l}, \hat{j}\right) = \underset{l,j}{\arg\min} \, \mathrm{Num}\!\left(\boldsymbol{F}_{l,t,j} = NaN\right) \qquad (1\,2)$$

[Math. 13]

$$\left(\hat{\hat{l}}, \hat{\hat{j}}\right) = \underset{\hat{l},\hat{j}}{\arg\min} \, \mathrm{Var}\!\left(\boldsymbol{F}_{\hat{l},t,\hat{j}}\right) \qquad (1\,3)$$

In expressions (12) and (13) above, Num indicates the number of elements. Var indicates the variance.

In an embodiment, the electronic device 1 can use the above processing to obtain the optimum heartbeat frequency vector as indicated by the following expression (14).

[Math. 14]

$$\boldsymbol{f}^{hs} = \boldsymbol{F}_{\hat{l},t,\hat{j}} \qquad (1\,4)$$

In an embodiment, the electronic device 1 can use the above processing to obtain a heart rate data vector r by taking the reciprocal of the optimum heartbeat frequency vector as indicated by expression (14) and then performing integer conversion processing, as indicated by the following expression (15).

[Math. 15]

$$\boldsymbol{r} = \mathrm{round}\!\left(\frac{1}{\boldsymbol{f}^{hs}}\right) \qquad (1\,5)$$

Here, round indicates an operation of rounding to the nearest integer.

In an embodiment, the electronic device 1 can perform processing like the above-described to obtain a heart sound time-series waveform and also obtain a heart rate time-series waveform.

Consequently, in step S25 illustrated in FIG. 13, the signal processing unit 10 can generate a heart rate time-series vector.

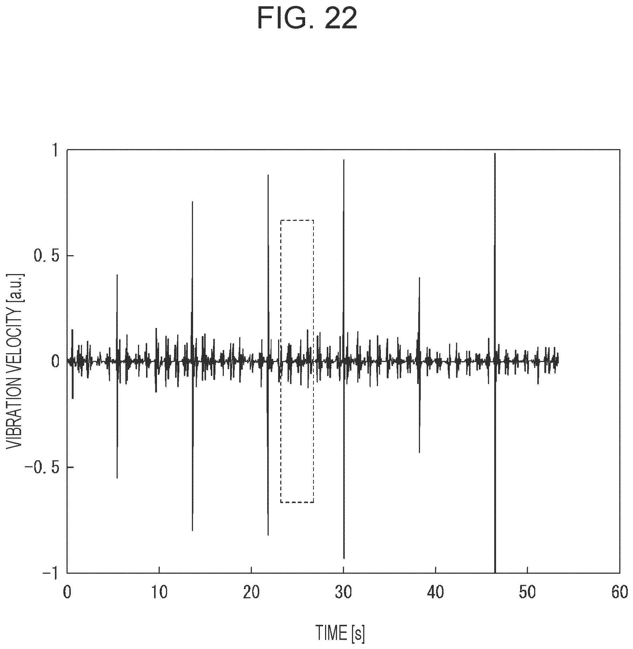

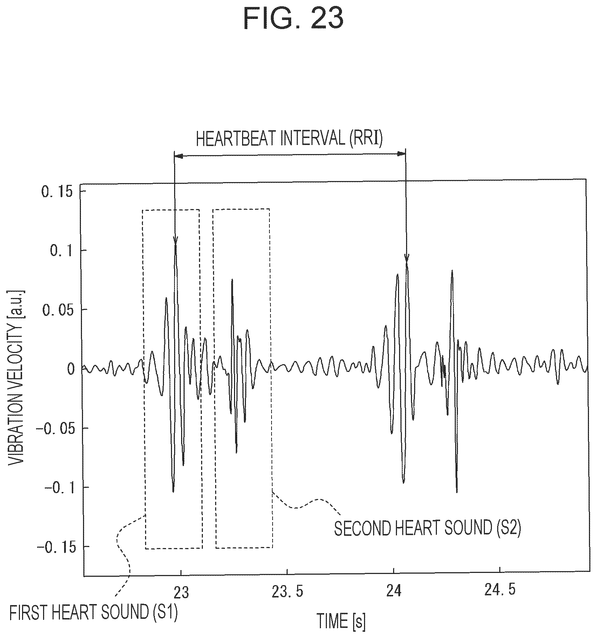

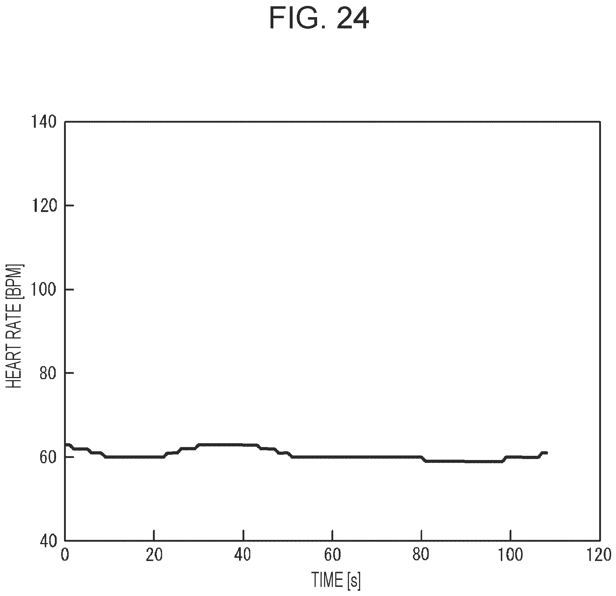

FIGs. 22, 23, and 24 illustrate examples of data obtained by the electronic device 1 according to an embodiment. FIGs. 22 and 23 illustrate an example of a heart sound time-series waveform obtained by the electronic device 1 according to an embodiment. FIG. 24 illustrates an example of a heart sound time-series waveform obtained by the electronic device 1 according to an embodiment. In FIGs. 22 and 23, the horizontal axis represents time, and the vertical axis represents vibration velocity. In FIG. 24, the horizontal axis represents time, and the vertical axis represents heart rate. FIG. 22 illustrates a heart sound time-series waveform obtained by the electronic device 1 according to an embodiment. FIG. 23 illustrates an enlarged view of the timespan in the region enclosed by the dashed lines in FIG. 22.

As illustrated in FIG. 23, the first heart sound S1 and the second heart sound S2 can be identified clearly from the heart sound time-series waveform obtained by the electronic device 1 according to an embodiment. As illustrated in FIG. 23, the heartbeat interval RRI calculated from the interval between the first heart sound S1 and the second heart sound S2 can also be identified clearly from the heart sound time-series waveform obtained by the electronic device 1 according to an embodiment. Strictly speaking, the RRI and the heartbeat interval are not the same, but are considered to approximately match. Accordingly, in the present disclosure, the RRI and the heartbeat interval are described as indicating the same thing.

FIG. 24 illustrates an example heart rate time-series data calculated from the heart sound time-series data illustrated in FIGs. 22 and 23. The result illustrated in FIG. 24 is calculated on the basis of the accurately extracted heart sound in FIGs. 22 and 23.

As described above, according to an embodiment, the electronic device 1 can obtain a detailed heart sound waveform as illustrated in FIGs 26 and 27, accurate RRI time-series data as illustrated in FIG. 28, and the power spectral density (PSD) of the heartbeat interval as illustrated in FIG. 29, for example. According to an embodiment, the electronic device 1 may detect the heartbeat of a human body or the like with good precision by transmitting and receiving radio waves. Consequently, according to an embodiment, the electronic device 1 can detect weak vibrations, such as the heartbeat of a human body, with good precision by transmitting and receiving radio waves, such as millimeter waves for example, and is anticipated to be useful in a wide variety of fields.

According to an embodiment, the electronic device 1 can extract heart sound by radar using a high-frequency band at or above millimeter waves and the analyze the heart sound itself to achieve accurate heartbeat interval extraction. To obtain a highly precise and robust heart rate, it may be necessary to search the space of data formed by the radar data appropriately to estimate the heart rate. According to an embodiment, the electronic device 1 can acquire the heart rate in a highly precise and robust way.

In the examples in FIGs. 22 through 24, only the first heart sound S1 and second heart sound S2 prominent in healthy subjects are described. However, according to an embodiment, the electronic device 1 can also perform processing in a similar manner in the case of a third heart sound and/or fourth heart sound occurring as a result of an abnormal heartbeat.

(Other embodiments)

The following describes other embodiments.

According to the embodiment described above, the electronic device 1 executes processing according to the MUSIC algorithm in step S24 of FIG. 13. However, in another embodiment, the electronic device 1 may also execute processing according to another subspace method, such as the Root-MUSIC algorithm, in step S24 of FIG. 13.

In [Processing A] described in step S24 of FIG. 13, a plurality of heartbeat frequency classes and a plurality of heart rate classes are set. However, in another embodiment, one class may be set as the heartbeat frequency class and the heart rate class if user heart rate ranges are stored in a database or the like and known. In this case, the heartbeat frequency data tensor is simplified to $F_{l,t,j} = F_{l,t}$. Consequently, in this case, the only index to be narrowed down by expressions (12) and (13) above is the index l of the window functions on the 2D-FFT.

In another embodiment, the processing in step S14 illustrated in FIG. 12 and/or the processing in step S21 illustrated in FIG. 13 may be omitted, depending on the situation. In another embodiment, the processing in step S14 (singular value decomposition/processing to analyze principal components) illustrated in FIG. 12 may be substituted with another subspace method.

Another embodiment describes a case in which [Processing A] and [Processing B] process heartbeat frequency vectors in step S24 illustrated in FIG. 13. However, in another embodiment, processing to convert the heartbeat frequency vectors into heart rate vectors may also be performed prior to [Processing A].

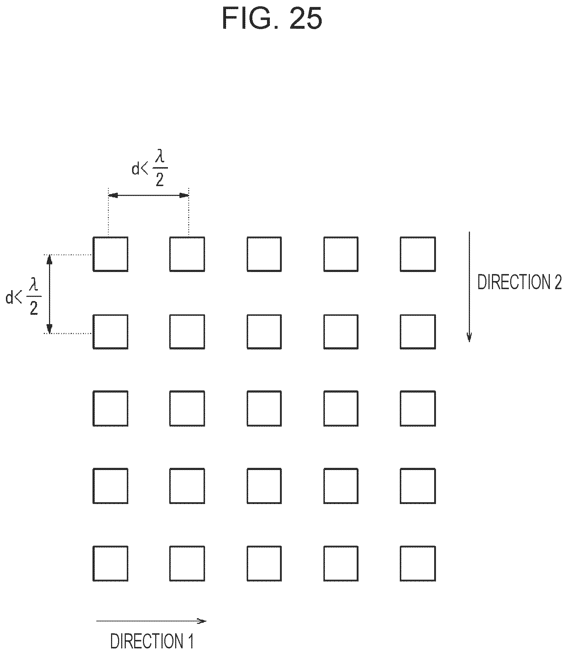

In an embodiment, the transmitting antenna 24 and/or receiving antenna 31 provided in the electronic device 1 is not limited to an arrangement like the one illustrated in FIG. 8. For example, in an embodiment, the receiving antenna 31 provided in the electronic device 1 may have a configuration like the one illustrated in FIG. 25. FIG. 30 illustrates an example of a uniform rectangular array (URA) receiving antenna. A URA receiving antenna like the one illustrated in FIG. 30 may be adopted to perform direction-of-arrival estimation for two angles by the URA receiving antenna alone, without using a beamformer to change the directivity in the transmitting antenna array 24.

In the electronic device 1 illustrated in FIG. 2, the signal processing unit 10 is described as being provided with functional units such as the heartbeat extraction unit 13 and the calculation unit 14. However, in an embodiment, the processing performed by the heartbeat extraction unit 13 and/or the calculation unit 14 may also be performed by an external computer, processor, or the like.

In an embodiment, the processing in step S14 (singular value decomposition/processing to analyze principal components) illustrated in FIG. 12 may be omitted if the number of samples N of one chirp can be set to a large number. In an embodiment, the processing in step S14 (singular value decomposition/processing to analyze principal components) illustrated in FIG. 12 may also be omitted if the mixing of other noise can be eliminated by a hardware-based method or the like.

In the processing in step S23 (generate heart sound envelope waveform) illustrated in FIG. 13, the result of the continuous wavelet transform is calculated by summation in the frequency-axis direction according to [Pseudocode 2]. However, the envelope waveform may also be generated by another technique, such as a moving average or the Hilbert transform.

As described above, in an embodiment, the electronic device 1 uses, for example, a millimeter-wave sensor provided with a plurality of transmitting antennas and a plurality of receiving antennas to detect weak vibrations such as a heartbeat. In an embodiment, when the subject is not detected, the electronic device 1 detects the body motion of the subject while varying the transmission phase of the antennas to create a beamforming pattern of the transmitting antennas. On the other hand, in an embodiment, once the body motion of the subject is detected, the electronic device 1 carries out beamforming in the direction of the body motion to detect the heartbeat. In this way, according to an embodiment, the electronic device 1 can improve the signal quality by automatically detecting the direction of a human body. Consequently, according to an embodiment, the electronic device 1 can improve the heartbeat detection precision and/or detection range. Thus, according to an embodiment, the electronic device 1 can detect the heartbeat of a human being with high precision.

The present disclosure has been described on the basis of the drawings and examples, but note that a person skilled in the art could easily make various variations or revisions on the basis of the present disclosure. Consequently, it should be understood that these variations or revisions are included in the scope of the present disclosure. For example, the functions and the like included in each functional unit may be rearranged in logically non-contradictory ways. Multiple functional units or the like may be combined into one, or a functional unit may be divided. Each embodiment according to the present disclosure described above is not limited to being carried out exactly according to each embodiment as described, and may be carried out by combining features or omitting some features, as appropriate. In other words, the content of the present disclosure enables a person skilled in the art to make various variations and revisions on the basis of the present disclosure. Therefore, these variations and revisions are included in the scope of the present disclosure. For example, in each embodiment, each functional unit, means, step, and the like can be added to another embodiment or replaced by each functional unit, means, step, and the like of another embodiment in logically non-contradictory ways. In each embodiment, multiple functional units, means, steps, and the like can be combined into one, or each functional unit, means, step, and the like can be divided. Each embodiment according to the present disclosure described above is not limited to being carried out exactly according to each embodiment as described, and can be carried out by combining features or omitting some features, as appropriate.

The embodiments described above are not limited solely to embodiments of the electronic device 1. For example, the embodiments described above may also be carried out as a method of controlling a device like the electronic device 1. Furthermore, the embodiments described above may also be carried out as a program to be executed by a device like the electronic device 1, for example, or as a storage medium or recording medium in which the program is recorded.

In the embodiments described above, the electronic device 1 is described as including components such as the transmitting antenna 24 and the receiving antenna 31 that form what is called a radar sensor. However, in an embodiment, the electronic device may be carried out as a configuration like the signal processing unit 10, for example. In this case, the signal processing unit 10 may be carried out as a unit having functions for processing signals handled by the transmitting antenna 24, the receiving antenna 31, and the like.

REFERENCE SIGNS

1      electronic device
10     signal processing unit
11     signal generation processing unit
12     received signal processing unit
13     heartbeat extraction unit
14     calculation unit
21     transmission DAC
22     transmission circuit
23     millimeter-wave transmission circuit
24     transmitting antenna
31     receiving antenna
32     mixer
33     reception circuit
34     reception ADC
50     communication interface
60     external device

**Claims**

1.  An electronic device comprising:

    a transmission unit configured to transmit a transmission wave;
    a reception unit configured to receive a reflected wave of the transmission wave from a target; and
    a signal processing unit configured to detect a distance, direction, and velocity of the target on the basis of a converted signal obtained by taking the Fourier transform of a beat signal of the transmission wave and the received wave, wherein
    the signal processing unit is configured to

    extract, by using a plurality of window functions, signal components corresponding to heart sound vibration associated with the heartbeat of the target from the converted signal,
    perform frequency analysis on an envelope signal obtained by performing envelope processing on the signal components corresponding to the heart sound vibration,
    perform processing to calculate a statistical result on the result of the frequency analysis, and
    output time-series data on the frequency of the heartbeat of the target on the basis of the calculated result.

2. The electronic device according to claim 1, wherein the signal processing unit is configured to perform super-resolution frequency analysis as the frequency analysis.

3. The electronic device according to claim 1, wherein the signal processing unit is configured to perform analysis based on a subspace method as the frequency analysis.

4. The electronic device according to claim 3, wherein the signal processing unit is configured to perform analysis based on the MUSIC algorithm as the frequency analysis.

5. A method of controlling an electronic device, the method comprising:

transmitting a transmission wave from a transmission unit;
a reception unit that receives a reflected wave of the transmission wave from a target;
detecting a distance position, direction, and velocity of the target on the basis of a converted signal obtained by taking the Fourier transform of a beat signal of the transmission wave and the received wave;
extracting, by using a plurality of window functions, signal components corresponding to heart sound vibration associated with the heartbeat of the target from the converted signal;
performing frequency analysis on an envelope signal obtained by performing envelope processing on the signal components corresponding to the heart sound vibration;
performing processing to calculate a statistical result on the result of the frequency analysis, and
outputting time-series data on the frequency of the heartbeat of the target on the basis of the calculated result.

6. A program causing an electronic device to execute a process comprising:

transmitting a transmission wave from a transmission unit;
a reception unit that receives a reflected wave of the transmission wave from a target;
detecting a distance position, direction, and velocity of the target on the basis of a converted signal obtained by taking the Fourier transform of a beat signal of the transmission wave and the received wave;
extracting, by using a plurality of window functions, signal components corresponding to heart sound vibration associated with the heartbeat of the target from the converted signal;
performing frequency analysis on an envelope signal obtained by performing envelope processing on the signal components corresponding to the heart sound vibration;
performing processing to calculate a statistical result on the result of the frequency analysis, and
outputting time-series data on the frequency of the heartbeat of the target on the basis of the calculated result.

# FIG. 1

# FIG. 2

EP 4 589 337 A1

# FIG. 3

EP 4 589 337 A1

# FIG. 4

SUBFRAME 1

SUBFRAME N

# FIG. 5

VELOCITY

s1

s2

DISTANCE

# FIG. 6

FIG. 7

# FIG. 8

# FIG. 9

EP 4 589 337 A1

FIG. 10

# FIG. 11

```
           ┌──────────────┐
           │    START     │
           └──────┬───────┘
                  │
                  ▼
    ┌─────────────────────────────┐
    │   PROCESS RECEIVED SIGNAL    │ ⟶ S110
    └──────────────┬──────────────┘
                   │
                   ▼
    ┌─────────────────────────────┐
    │    EXTRACT VIBRATING SOURCE  │ ⟶ S120
    └──────────────┬──────────────┘
                   │
                   ▼
    ┌─────────────────────────────┐
    │  CONVERT TO VIBRATION WAVEFORM │ ⟶ S130
    └──────────────┬──────────────┘
                   │
                   ▼
    ┌─────────────────────────────┐
    │     EXTRACT VIBRATION DATA   │ ⟶ S140
    └──────────────┬──────────────┘
                   │
                   ▼
    ┌─────────────────────────────┐
    │        CALCULATE RRI         │ ⟶ S150
    └──────────────┬──────────────┘
                   │
                   ▼
    ┌─────────────────────────────┐
    │        CALCULATE HRV         │ ⟶ S160
    └──────────────┬──────────────┘
                   │
                   ▼
           ┌──────────────┐
           │     END      │
           └──────────────┘
```

# FIG. 12

```
        ┌─────────────┐
        │    START    │
        └─────────────┘
               │
               ▼
  ┌──────────────────────────────┐
  │   PROCESS RECEIVED SIGNAL     │──── S11
  └──────────────────────────────┘
               │
               ▼
  ┌──────────────────────────────┐
  │ PERFORM MULTI-WINDOW PROCESSING│──── S12
  └──────────────────────────────┘
               │
               ▼
  ┌──────────────────────────────┐
  │    RECONSTRUCT SIGNAL SET     │──── S13
  └──────────────────────────────┘
               │
               ▼
  ┌──────────────────────────────┐
  │   PERFORM PRINCIPAL COMPONENT │──── S14
  │   ANALYSIS/NOISE REMOVAL      │
  └──────────────────────────────┘
               │
               ▼
  ┌──────────────────────────────┐
  │   CONVERT TO VIBRATION WAVEFORM│──── S15
  └──────────────────────────────┘
               │
               ▼
  ┌──────────────────────────────┐
  │ ANALYZE HEART SOUND/HEARTBEAT INTERVAL │──── S16
  └──────────────────────────────┘
               │
               ▼
        ┌─────────────┐
        │     END     │
        └─────────────┘
```

# FIG. 13

START

↓

REMOVE NOISE — S21

↓

EXTRACT HEART SOUND — S22

↓

GENERATE ENVELOPE WAVEFORM — S23

↓

ANALYZE HEARTBEAT FREQUENCY — S24

↓

GENERATE TIME-SERIES VECTOR OF HEART RATE — S25

↓

END

FIG. 14

# FIG. 15

# FIG. 16

$c_{3,k}$

$\cdots$

$c_{2,k}$

$d_{3,k}$

$\cdots$

$c_{1,k}$

$d_{2,k}$

LEVEL 2 DETAIL
COEFFICIENTS

$v^{I}_{vib, dn}$

$d_{1,k}$

LEVEL 1 DETAIL
COEFFICIENTS

FIG. 17

# FIG. 18

# FIG. 19

# FIG. 20

# FIG. 21

# FIG. 22

# FIG. 23

# FIG. 24

# FIG. 25

$$d < \frac{\lambda}{2}$$

$$d < \frac{\lambda}{2}$$

DIRECTION 2

DIRECTION 1

## INTERNATIONAL SEARCH REPORT

| International application No. |
|---|
| **PCT/JP2023/033445** |

| A. | CLASSIFICATION OF SUBJECT MATTER |
|---|---|

*G01S 13/34*(2006.01)i; *A61B 5/0245*(2006.01)i; *A61B 5/11*(2006.01)i; *G01S 13/88*(2006.01)i
FI:   G01S13/34; A61B5/0245 100Z; A61B5/11 110; G01S13/88

According to International Patent Classification (IPC) or to both national classification and IPC

| B. | FIELDS SEARCHED |
|---|---|

Minimum documentation searched (classification system followed by classification symbols)

G01S7/00-7/42, 13/00-13/95; A61B5/0245; A61B5/11

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); Scopus

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2021-1735 A (ASAHI KASEI DENSHI KK) 07 January 2021 (2021-01-07)<br>entire text, all drawings | 1-6 |
| A | CN 113261925 A (SHANDONG NORMAL UNIVERSITY) 17 August 2021 (2021-08-17)<br>entire text, all drawings | 1-6 |
| A | CN 112401856 A (WUHAN FENGHUO KAIZHUO TECHNOLOGY CO., LTD.) 26 February 2021 (2021-02-26)<br>entire text, all drawings | 1-6 |
| A | JP 2017-127398 A (KEIO GIJUKU) 27 July 2017 (2017-07-27)<br>entire text, all drawings | 1-6 |
| A | JP 2021-176426 A (UNIV OF KITAKYUSHU) 11 November 2021 (2021-11-11)<br>entire text, all drawings | 1-6 |
| A | US 2018/0279884 A1 (TEXAS INSTRUMENTS INCORPORATED) 04 October 2018 (2018-10-04)<br>entire text, all drawings | 1-6 |

☑ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **04 October 2023** | **24 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
|---|
| **PCT/JP2023/033445** |

### C. DOCUMENTS CONSIDERED TO BE RELEVANT

| Category* | Citation of document, with indication, where appropriate, of the relevant passages | Relevant to claim No. |
|---|---|---|
| A | US 2019/0328243 A1 (EMORY UNIVERSITY) 31 October 2019 (2019-10-31) entire text, all drawings | 1-6 |

Form PCT/ISA/210 (second sheet) (January 2015)

**INTERNATIONAL SEARCH REPORT**
Information on patent family members

International application No.

**PCT/JP2023/033445**

| Patent document cited in search report | | | Publication date (day/month/year) | Patent family member(s) | Publication date (day/month/year) |
|---|---|---|---|---|---|
| JP | 2021-1735 | A | 07 January 2021 | (Family: none) | |
| CN | 113261925 | A | 17 August 2021 | (Family: none) | |
| CN | 112401856 | A | 26 February 2021 | (Family: none) | |
| JP | 2017-127398 | A | 27 July 2017 | (Family: none) | |
| JP | 2021-176426 | A | 11 November 2021 | (Family: none) | |
| US | 2018/0279884 | A1 | 04 October 2018 | (Family: none) | |
| US | 2019/0328243 | A1 | 31 October 2019 | WO 2018/119316 A1 entire text, all drawings EP 3895605 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022148633 A
- JP 2002071825 A
- JP 2021032880 A